# EUROPEAN PATENT APPLICATION

(11) **EP 1 195 433 A2**
(43) Date of publication of application: **10.04.2002**
(21) Application number: 01121110.9
(22) Date of filing: 03.09.2001
(51) Int. Cl.: C12N 9/02, C12N 15/53

(54) **Respiratory chain enzyme genes of coryneform bacteria**

(30) Priority: 06.09.2000 JP 2000270283
(71) Applicant: Ajinomoto Co., Inc., Tokyo (JP)
(72) Inventor: Sone, Nobuhito, Iizuka-shi, Fukuoka (JP)
(74) Representative: Strehl Schübel-Hopf & Partner

(57) **Abstract**

A gene coding for aa3 subunit I is obtained from a *Corynebacterium glutamicum* chromosomal DNA library by hybridization utilizing a probe produced based on a sequence common to a wide range of heme-copper enzymes. A gene coding for subunit II and a gene coding for subunit III are obtained by hybridization using a probe produced by using primers prepared based on the N-terminus amino acid sequence of subunit II, which is determined by using purified cytochrome aa3, and an operon coding for cytochrome bcl complex existing downstream from the foregoing genes is further obtained.

## Description

### Background of the Invention

### Field of the Invention

The present invention relates to respiratory chain enzymes of coryneform bacteria, more precisely enzymes constituting the electron transport system, and genes coding for the enzymes.

### Description of the Related Art

Most of organisms acquire energy necessary for life activity by respiration. In higher organisms, carbohydrates, proteins, and aliphatic acids are degraded into acetyl-CoA by the glycolytic pathway and the β-oxidation in cytoplasm, and acetyl-CoA is degraded by the citric acid cycle in mitochondria. The resulting energy is saved as reducing power of NADH and FADH₂. Finally, NADH is completely oxidized to water by the subsequent electron transport system that is present on mitochondrial inner membranes, and a proton concentration gradient is formed in a coupled manner to the oxidation, and serves as driving force of the ATP synthesis.

Since the bacterial respiratory chain generally comprises various functional enzyme complexes depending on species and growing circumstance, the energy conservation efficiency may vary to a great extent. For example, *Escherichia coli* contains at least two kinds of quinol oxidases, bo type and bd type, which function as terminal oxidases in the respiratory chain. When a wild-type strain carrying the enzymes of the both types, a mutant strain carrying only the bo type, and a mutant strain carrying only the bd type are compared as for growth yield observed in aerobic culture, the growth yield is the lowest in the mutant carrying only the bd type enzyme, and depends on the kind of the terminal oxidases and their energy conservation efficiency (Lecture Abstract for The Conference of The Society for Bioscience and Bioengineering, Japan, 1995, Subject No. 357).

Coryneform bacteria such as *Brevibacterium lactofermentum* and *Brevibacterium flavum* are gram-positive and aerobic bacteria that are industrially utilized for amino acid producers. Although terminal oxidases of the respiratory chain have been well investigated as for those of *Proteobacteria,* which is phylogenetically quite far from the coryneform bacteria, and those of *Bacillus subtilis* and the thermophilic *Bacillus,* which are also gram-positive bacteria like the coryneform bacteria but phylogenetically somewhat different from them, the electron transport system of respiratory chain in coryneform bacteria has not been investigated in detail. It is considered that it is important to elucidate the electron transport system of the respiratory chain, which is the key of the energy metabolism, in coryneform bacteria in view of collecting fundamental data for improving productivity of useful substances. Further, if enzymes involved in the electron transport system of the respiratory chain in coryneform bacteria and genes therefor are identified, they may be useful for, for example, creating strains with higher energy efficiency.

Thus far, it has been reported that the respiration of *Brevibacterium lactofermentum* is coupled to the proton transport, and it involves cytochromes a, b and c (Kawahara, Y., *et al., Agric. Biol. Chem., 52* (8), 1979-1983 (1988)), and further a gene coding for a cytochrome bd type quinol oxidase has been isolated (Japanese Patent Laid-open Publication (Kokai) No. 11-346776, Europe Patent Laid-open Publication 0 967 282(A2)).

Further, in *Corynebacterium glutamicum,* there is a cytochrome bc1 complex, and presence of at least two kinds of terminal oxidases, SoxM type oxidase and cytochrome bd type oxidase, is confirmed (The Second Symposium Concerning Metabolic Engineering, Lecture Abstracts, 1999). This shows that the electron transfer pathway form quinone pool to oxygen molecule include two kinds of pathways, a pathway utilizing cytochrome bc1 complex and SoxM type oxidase (Castresana J, Saraste M., *Trends in Biochem. Sci., 20,* 443-448 (1995)) and a pathway utilizing only the cytochrome bd type oxidase. It is considered that the former is an electron transfer pathway of high energy efficiency, in which proton translocation value for transfer of one electron is high, and the latter is an electron transfer pathway of low energy efficiency, in which proton translocation value for transfer of one electron is low.

By the way, a cytochrome aa3 type oxidase is structurally defined as a heme-copper oxidase, and classified into SoxM type oxidases. It is known that there are two kinds of SoxM type oxidases, *i.e.,* cytochrome c oxidase and quinol oxidase, and they have high proton transportation ability. Oxidases showing homology to the cytochrome aa3 type oxidase already discovered in microorganisms are known for *Paracoccus denitrificans, Bradyrhizobium japonicum, Rhodobacter sphaeroides, Synechococcus vulcanus, Thermus thermophilus, Bacillus subtilis, Bacillus stearothermophilus* and so forth (Trumpower, B.L. and Gennis, R.B., *Annu. Rev. Biochem., 63,* 675-716 (1994); Cao, J. *et al., J. Biol. Chem., 267,* 24273-24278 (1992); Sone, N. *et al., Biochim. Biophys. Acta., 1183,* 130-138 (1993); Sakamoto, J. *et al., J. Biochem., 122,* 764-771 (1997)).

On the other hand, the cytochrome bc1 complexes widely exist in respiratory chains of various organisms, such as those in mitochondria and microorganisms, and constitute a superfamily. These enzymes play an important role in the production of energy required for organisms, that is, they transport electrons from quinol to cytochrome c, and simultaneously, pump out protons form the inside of membrane to the outside of membrane to form a transmembrane proton concentration gradient. Further, these cytochrome c1 reductases are considered to transport protons form the inside of membrane to the outside of membrane by a mechanism called proton motive Q cycle (Mitchell, P., *J. Theoret. Biol.,* 62, 327-367 (1976); Croft, A.R., Meinhardt, S.W., Jones, K.R., and Snozzi, *M., Biochim. Biophys. Acta, 723,* 202-218 (1983); Trumpower, B.L., *J. Biol. Chem., 265,* 11409-11412 (1990)). The cytochrome bc1 complexes are known for *Paracoccus denitrificans, Rhodobacter sphaeroides* and *Bacillus stearothermophilus* (Trumpower B.L. and Gennis RB., *Annu. Rev. Biochem., 63,* 675-716 (1994), Sone *et al., J. Biol. Chem., 271,* 12457-12462 (1996)).

### Summary of the Invention

An object of the present invention is to provide a cytochrome aa3 type oxidase and cytochrome bc1 complex of coryneform bacteria, as well as genes coding for them.

In order to achieve the aforementioned object, the inventors of the present invention attempted to clone a cytochrome aa3 gene of *Corynebacterium glutamicum*. As a result, they successfully cloned a gene coding for the aa3 subunit I (also referred to as *"ctaD"* hereinafter) from a chromosomal DNA library of *Corynebacterium glutamicum* by hybridization utilizing a probe produced based on a sequence common to a wide range of heme-copper oxidases. However, unlike many other oxidases, genes of other subunits were not found in the neighborhood of that gene.

On the other hand, the inventors of the present invention also successfully purified cytochrome aa3 from a membrane preparation of *Corynebacterium glutamicum,* and they isolated a subunit II protein from that purified enzyme, and determined its N-terminus partial peptide sequence. A primer was prepared based on this sequence, and PCR was performed by using this primer and a primer prepared based on the conserved sequence of CuA binding motif in the oxidase subunit II to produce a probe. Then, hybridization was performed by using this probe to successfully obtain a gene coding for the subunit II (also referred to as *"ctaC"* hereinafter). Furthermore, it was also found that a gene coding for the subunit III (also referred to as *"ctaE"* hereinafter) existed downstream from that gene.

Further, a sequence estimated to be a part of ORF was found at a position on the 3' end side and downstream from the aforementioned gene coding for the cytochrome aa3 subunit III (*ctaE*). Then, a clone containing this sequence was obtained and its sequence was determined. Furthermore, a subunit containing cytochrome c among the subunits constituting the cytochrome bc1 complex was purified, and its amino acid sequence was determined. As a result, it was found that the aforementioned clone contained an operon coding for the three subunits of cytochrome bc1 complex (*qcrCAB*).

The present invention was accomplished as described above, and provides the followings.
(1) A polypeptide defined in the following (A1) or (A2):
   (A1) a polypeptide that has the amino acid sequence of SEQ ID NO: 2,
   (A2) a polypeptide that has the amino acid sequence of SEQ ID NO: 2 comprising substitution, deletion, insertion or addition of one or several amino acid residues in the amino acid sequence, and can constitute cytochrome aa3 together with a cytochrome aa3 subunit II having the amino acid sequence of SEQ ID NO: 4 and cytochrome aa3 subunit III having the amino acid sequence of SEQ ID NO: 5.
(2) A polypeptide defined in the following (B1) or (B2):
   (B1) a polypeptide that has the amino acid sequence of SEQ ID NO: 4,
   (B2) a polypeptide that has the amino acid sequence of SEQ ID NO: 4 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit III having the amino acid sequence of SEQ ID NO: 5.
(3) A polypeptide defined in the following (C1) or (C2):
   (C1) a polypeptide that has the amino acid sequence of SEQ ID NO: 5,
   (C2) a polypeptide that has the amino acid sequence of SEQ ID NO: 5 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit II having the amino acid sequence of SEQ ID NO: 4.
(4) A cytochrome aa3 consisting of the polypeptides according to (1), (2) and (3).
(5) A polypeptide defined in the following (D1) or (D2):
   (D1) a polypeptide that has the amino acid sequence of SEQ ID NO: 7,
   (D2) a polypeptide that has the amino acid sequence of SEQ ID NO: 7 comprising substitution, deletion, insertion or addition of one or several amino acid residues in the amino acid sequence, and can constitute a cytochrome bc1 complex together with QcrA having the amino acid sequence of SEQ ID NO: 8 and QcrB having the amino acid sequence of SEQ ID NO: 10.
(6) A polypeptide defined in the following (E1) or (E2):
   (E1) a polypeptide that has the amino acid sequence of SEQ ID NO: 8,
   (E2) a polypeptide that has the amino acid sequence of SEQ ID NO: 8 comprising substitution, deletion, insertion or addition of one or several amino acid residues in the amino acid sequence, and can constitute a cytochrome bc1 complex together with QcrC having the amino acid sequence of SEQ ID NO: 7 and QcrB having the amino acid sequence of SEQ ID NO: 10.
(7) A polypeptide defined in the following (F1) or (F2):
   (F1) a polypeptide that has the amino acid sequence of SEQ ID NO: 10,
   (F2) a polypeptide that has the amino acid sequence of SEQ ID NO: 10 comprising substitution, deletion, insertion or addition of one or several amino acid residues in the amino acid sequence, and can constitute a cytochrome bc1 complex together with QcrC having the amino acid sequence of SEQ ID NO: 7 and QcrA having the amino acid sequence of SEQ ID NO: 8.
(8) A cytochrome bc1 complex consisting of the polypeptides according to (5), (6) and (7).
(9) A DNA coding for a polypeptide defined in the following (A1) or (A2):
   (A1) a polypeptide that has the amino acid sequence of SEQ ID NO: 2,
   (A2) a polypeptide that has the amino acid sequence of SEQ ID NO: 2 comprising substitution, deletion, insertion or addition of one or several amino acid residues in the amino acid sequence, and can constitute cytochrome aa3 together with a cytochrome aa3 subunit II having the amino acid sequence of SEQ ID NO: 4 and cytochrome aa3 subunit III having the amino acid sequence of SEQ ID NO: 5.
(10) A DNA coding for a polypeptide defined in the following (B1) or (B2):
   (B1) a polypeptide that has the amino acid sequence of SEQ ID NO: 4,
   (B2) a polypeptide that has the amino acid sequence of SEQ ID NO: 4 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit III having the amino acid sequence of SEQ ID NO: 5.
(11) A DNA coding for a polypeptide defined in the following (C1) or (C2):
   (C1) a polypeptide that has the amino acid sequence of SEQ ID NO: 5,
   (C2) a polypeptide that has the amino acid sequence of SEQ ID NO: 5 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit II having the amino acid sequence of SEQ ID NO: 4.
(12) A DNA coding for a polypeptide defined in the following (D1) or (D2):
   (D1) a polypeptide that has the amino acid sequence of SEQ ID NO: 7,
   (D2) a polypeptide that has the amino acid sequence of SEQ ID NO: 7 comprising substitution, deletion, insertion or addition of one or several amino acid residues in the amino acid sequence, and can constitute a cytochrome bc1 complex together with QcrA having the amino acid sequence of SEQ ID NO: 8 and QcrB having the amino acid sequence of SEQ ID NO: 10.
(13) A DNA coding for a polypeptide defined in the following (E1) or (E2):
   (E1) a polypeptide that has the amino acid sequence of SEQ ID NO: 8,
   (E2) a polypeptide that has the amino acid sequence of SEQ ID NO: 8 comprising substitution, deletion, insertion or addition of one or several amino acid residues in the amino acid sequence, and can constitute a cytochrome bc1 complex together with QcrC having the amino acid sequence of SEQ ID NO: 7 and QcrB having the amino acid sequence of SEQ ID NO: 10.
(14) A DNA coding for a polypeptide defined in the following (F1) or (F2):
   (F1) a polypeptide that has the amino acid sequence of SEQ ID NO: 10,
   (F2) a polypeptide that has the amino acid sequence of SEQ ID NO: 10 comprising substitution, deletion, insertion or addition of one or several amino acid residues in the amino acid sequence, and can constitute a cytochrome bc1 complex together with QcrC having the amino acid sequence of SEQ ID NO: 7 and QcrA having the amino acid sequence of SEQ ID NO: 8.

In the present invention, the expression that "a polypeptide can constitute cytochrome aa3" means that the polypeptide has a property that it can form a protein complex that shows oxidation-reduction spectrum of heme a, CO binding reduction-type minus reduction-type difference spectrum of heme a3, and an activity for receiving an electron from cytochrome c and transmitting the electron to oxygen so as to reduce it to a water molecule, together with the other subunit peptides. Further, the expression that "a polypeptide can constitute cytochrome bc1 complex" means that the polypeptide has a property that it can form a protein complex that shows oxidation-reduction absorption spectra of heme b and heme c, and an activity for receiving an electron from a reduced type quinone compound (quinol) and transmitting the electron to cytochrome c or a terminal oxidase, together with the other subunit peptides.

According to the present invention, there are provided subunits constituting cytochrome aa3 and DNA coding for them. There are also provided subunits constituting cytochrome bc1 complex and DNA coding for them. These polypeptides and DNA are useful for elucidation of the electron transport system of coryneform bacteria. Further, the DNA of the present invention can be used for breeding of coryneform bacteria that produce useful substances with good energy efficiency.

### Brief Explanation of the Drawings

Fig. 1 shows structure of genes coding for the subunits of cytochrome aa3, and relationship between the structures and clones:
(A) structure of DNA fragment containing the *ctaC* gene coding for subunit II and the ctaE gene coding for the subunit III, and
(B) structure of DNA fragment containing *ctaD* coding for the subunit I.

Fig. 2 shows structures of genes coding for subunits of cytochrome bc1 complex (*qcrC, qcrA, qcrB*), and relationship between the structures and clones.

### Detailed Description of the Invention

Hereafter, the present invention will be explained in detail.

### <1> Cytochrome aa3, subunits thereof, and DNA coding for them

Among the DNAs coding for cytochrome aa3 of the present invention, the DNA coding for the subunit I can be obtained as follows. That is, primers are prepared from, for example, *Corynebacterium glutamicum* chromosomal DNA based an amino acid sequence of a region highly conserved in known heme-copper enzymes such as cytochrome c oxidases I of *Bradyrhizobium japonicum, Acetobacter aceti, Synechococcus vulcanus* and *Bacillus stearothermophilus,* and a probe is prepared by PCR using the primers and chromosomal DNA of *Corynebacterium glutamicum* as a template. Then, the intended DNA can be obtained from a *Corynebacterium glutamicum* chromosomal DNA library by hybridization using the probe obtained above.

The chromosomal DNA of *Corynebacterium glutamicum* can be prepared by, for example, the method of Saito and Miura (*Biochem. Biophys. Acta., 72*, 619, (1963)), or the method of K. S. Kirby (*Biochem. J., 64,* 405, (1956)). A chromosome DNA library can be obtained by partially digesting chromosomal DNA with a suitable restriction enzyme, ligating each of the obtained DNA fragments to a vector DNA autonomously replicable in *Escherichia coli* cell to prepare recombinant DNA, and introducing the DNA into *Escherichia coli.* The vector is not particularly limited, so long as it is a vector usually used for genetic cloning, and plasmid vectors such as pUC19, pUC18, pUC118 and pUC119, phage vectors such as lambda phage DNA and so forth can be used.

The primers used for the aforementioned PCR may be, for example, oligonucleotides having the nucleotide sequences of SEQ ID NO: 11 or SEQ ID NO: 12.

Screening of a chromosomal DNA library of *Corynebacterium glutamicum* utilizing a DNA fragment obtained in PCR as a probe can be performed by colony hybridization when plasmid vectors are used for the preparation of the library, or plaque hybridization when phage vectors are used for the preparation of the library. A hybridization positive clone can be confirmed if it contains the target gene coding for cytochrome aa3 subunit I *(ctaD)* by preparing DNA from the clone and determining its nucleotide sequence. It is also possible to preliminarily perform Southern analysis for a hybridization positive clone by using the aforementioned probe.

A nucleotide sequence of *ctaD* gene of *Corynebacterium glutamicum* KY9002 strain (ATCC13032) obtained in the working example to be mentioned later in such a manner as described above is shown in SEQ ID NO: 1. An expected coding region and amino acid sequence of protein encoded thereby are shown in SEQ ID NO: 1.

Further, genes coding for cytochrome aa3 subunits II and III (*ctaC* and *ctaE*) can be obtained as follows.

From a membrane preparation of *Corynebacterium glutamicum*, cytochrome aa3 is purified, and an N-terminus partial peptide sequence of each subunit is determined. A primer is prepared based on the sequence. By using this primer and a primer prepared based on a sequence highly conserved among the known oxidase subunits, for example, a conserved sequence of CuA binding motif in the subunit II, PCR is performed to produce a probe. Then, a DNA fragment containing the gene coding for the subunit II (*ctaC*) and the gene coding for the subunit III (*ctaE*) is obtained by hybridization using the probe.

The primers used for the aforementioned PCR may be, for example, oligonucleotides having the nucleotide sequences of SEQ ID NO: 14 or SEQ ID NO: 15.

A nucleotide sequence of a DNA fragment containing *ctaC* gene and *ctaE* gene of the *Corynebacterium* *glutamicum* KY9002 strain (ATCC13032), which was obtained in the working example to be mentioned later in the manner as described above, is shown in SEQ ID NO: 3. Expected coding regions and amino acid sequences of proteins encoded thereby are shown in SEQ ID NOS: 4 and 5. In the aforementioned DNA fragment, the *ctaC* gene and the *ctaE* gene were separate from each other by about 1 kb, and another ORF existed between them.

The numbers of amino acid residues of the subunits I, II and III of cytochrome aa3 are estimated to be 584, 316 and 205 residues, respectively, and molecular weights are calculated to be 65.0, 39.5 and 22.4 kDa, respectively. As for the subunit II, it is considered that an N-terminal sequence from first residue to the glycine residue at the 28th position is excised and the cysteine residue at the 29th position is modified with a lipid in a matured polypeptide in view of the results of the amino acid sequence analysis and analogy with oxidases of other species (*Bacillus subtilis* and *Escherlchia coli:* Santana, M. et *al., J. Biol. Chem., 267,* 10225-10231 (1992)).

In the amino acid sequence of the subunit II shown in SEQ ID NO: 4, the amino acid numbers 257-268 correspond to the conserved sequence of CuA binding motif.

In addition, the codons of the amino acid residues of the N-termini of the subunits II and III are GTG, and the corresponding amino acid residues are indicated as Val in Sequence Listing. However, they are actually Met, and it is considered that this is because GTG is recognized as an initiation methionine. Other examples of such a phenomenon have also been reported.

Analyses such as estimation of coding regions and operon structure can be performed by using GENETYX Homology Version 2.2.2 (Software Development Co., Ltd.). Homology analysis can be performed according to the method of Lipman and Peason *(Science, 227,* 1435-1441, 1985).

The nucleotide sequences of *ctaD, ctaC* and *ctaE* were elucidated by the present invention, and therefore these gene can be directly obtained by preparing primers based on the nucleotide sequence shown in SEQ ID NO: 1 or 3, and performing PCR utilizing them and *Corynebacterium glutamicum* chromosomal DNA as a template.

The DNA of the present invention may be one coding for either one of the aforementioned subunits I, II and III of cytochrome aa3, or one coding for two or three kinds of them. Cytochrome aa3 or a subunit thereof can be produced by introducing the DNA of the present invention into a suitable host cell, and culturing an obtained transformant to express the DNA. For example, the DNA coding for the subunit I may be a DNA having the nucleotide sequence consisting of nucleotide numbers 1538-3289 in the nucleotide sequence of SEQ ID NO: 1, the DNA coding for the subunit II may be a DNA having the nucleotide sequence consisting of nucleotide numbers 604-1680 in the nucleotide sequence of SEQ ID NO: 3, and the DNA coding for the subunit III may be a DNA having the nucleotide sequence consisting of nucleotide numbers 2715-3329 in the nucleotide sequence of SEQ ID NO: 3.

Produced cytochrome aa3 or subunits thereof can be collected and purified from culture by a method commonly used for the purification of proteins such as salting out, solvent precipitation, gel filtration chromatography and ion exchange chromatography.

The DNA of the present invention coding for the subunit I may be one coding for a polypeptide that has the amino acid sequence of SEQ ID NO: 2 comprising substitution, deletion, insertion or addition of one or several amino acid residues in the amino acid sequence, and can constitute cytochrome aa3 together with the subunit II and III.

The DNA of the present invention encoding the subunit II may be one coding for a polypeptide that has the amino acid sequence of SEQ ID NO: 4 comprising substitution, deletion, insertion or addition of one or several amino acid residues in the amino acid sequence, and can constitute cytochrome aa3 together with the subunit I and III.

The DNA of the present invention encoding the subunit III may be one coding for a polypeptide that has the amino acid sequence of SEQ ID NO: 5 comprising substitution, deletion, insertion or addition of one or several amino acid residues in the amino acid sequence, and can constitute cytochrome aa3 together with the subunit I and II.

Furthermore, a DNA coding for a cytochrome aa3 in which Subunit I, II or III, or two or three of them contain a mutation also falls within the scope of the DNA of the present invention.

The number of "several" amino acid residues is preferably 1-40, more preferably 1-10. Alternatively, the number is preferably such a number that the amino acid sequence should show homology of 80% or more, preferably 95% or more, to the amino acid sequence of SEQ ID NO: 2, 4 or 5.

Such a DNA coding for substantially the same protein as the subunit I, II or III as described above can be obtained by, for example, modifying each nucleotide sequence by, for example, the site-directed mutagenesis method so that the amino acid sequence should involve substitution, deletion, insertion or addition of one or more amino acid residues at a specified site. Such a DNA modified as described above may also be obtained by a conventionally known mutation treatment. The mutation treatment includes a method of treating DNA coding for the subunit I, II or III *in vitro,* for example, with hydroxylamine, and a method for treating a microorganism, for example, a bacterium belonging to the genus *Escherichia,* harboring a DNA coding for the subunit I and/or subunit II with ultraviolet irradiation or a mutating agent usually used for mutation treatment such as N-methyl-N'-nitro-N-nitrosoguanidine (NTG) and nitrous acid.

The substitution, deletion, insertion or addition of nucleotide as described above also includes a naturally occurring mutation (mutant or variant) on the basis of, for example, individual difference or difference in species or genus of coryneform bacteria that harbor cytochrome aa3 and so forth.

A DNA coding for substantially the same protein as the subunit I, II or III described above can be obtained by expressing such a DNA having a mutation as described above in a suitable cell, and examining the cytochrome aa3 activity of the expression product. A DNA coding for substantially the same protein as the subunit I, II or III can also be obtained by isolating a DNA hybridizable with a DNA having, for example, the nucleotide sequence corresponding to nucleotide numbers of 1538-3289 of the nucleotide sequence shown in SEQ ID NO: 1, the nucleotide sequence corresponding to nucleotide numbers of 604-1680 or the nucleotide numbers of 2715-3329 of the nucleotide sequence shown in SEQ ID NO: 3 under the stringent conditions, and coding for a protein having a function of the subunit I, II or III from a DNA coding for the subunit I, II or III including a mutation or a cell harboring it. The "stringent conditions" referred to herein is a condition under which so-called specific hybrid is formed, and nonspecific hybrid is not formed. It is difficult to clearly express this condition by using any numerical value. However, for example, the stringent conditions include a condition under which DNA's having high homology, for example, DNA's having homology of not less than 50%, are hybridized with each other, and DNA's having homology lower than the above level are not hybridized with each other. Alternatively, the stringent conditions are exemplified by a condition under which DNA's are hybridized with each other at a salt concentration corresponding to an ordinary condition of washing in Southern hybridization, *i.e., 1* x SSC, 0.1% SDS, preferably 0.1 x SSC, 0.1% SDS, at 60°C.

Genes hybridizable under such conditions as described above include those having a stop codon generated in the genes, and those no longer having activity. However, such genes can be readily removed by ligating each of the genes with a commercially available activity expression vector, and examining the function of the expression product.

The host for the expression of the DNA of the present invention include, for example, various kinds of bacteria including coryneform bacteria such as Escherichia *coli, Brevibacterium lactofermentum,* and *Brevibacterium flavum,* eucaryotic cells such as *Saccharomyces cerevisiae* and so forth. In order to introduce the DNA of the present invention into a host such as those mentioned above, the host cell can be transformed with a recombinant vector which is obtained by inserting the DNA of the present invention into a vector selected depending on the kind of the host in which the expression is to be obtained. Those procedures can be performed by using methods of genetic recombination well known to those skilled in the art.

Methods for preparation of chromosomal DNA, construction of chromosomal DNA library, hybridization, PCR, preparation of plasmid DNA, digestion and ligation of DNA, transformation, design of oligonucleotides used as primers and so forth are described in by Sambrook, J., Fritsch, E.F., Maniatis, T., "Molecular Cloning A Laboratory Manual, Second Edition", Cold Spring Harbor Laboratory Press (1989) and so forth.

The cytochrome aa3, subunits thereof and DNA coding for them of the present invention are considered to be useful for elucidation of the electron transport system of coryneform bacteria. Further, the DNA can be used for breeding of coryneform bacteria that produce useful substances with good energy efficiency.

### <2> Cytochrome bc1 complex, subunits thereof and DNA coding for them

A part of DNA coding for cytochrome bc1 complex was found in the DNA fragment containing the genes coding for cytochrome aa3 subunits II and III (*ctaC* and *ctaE)* obtained as described above, at a position downstream from the genes on the 3' side. Therefore, a DNA coding for cytochrome bc1 complex can be obtained by cloning the flanking regions of the *ctaE* gene of *Corynebacterium glutamicum* chromosomal DNA in a known manner.

Specifically, PCR is performed (95°C for 60 seconds, 52°C for 60 seconds, 68°C for 60 seconds, 35 cycles) by using the primers shown in SEQ ID NOS: 14 and 15 and chromosomal DNA of *Corynebacterium glutamicum* KY9002 strain (ATCC13032) as a template, and the obtained DNA fragment is used as a probe to screen a chromosomal DNA library by colony hybridization.

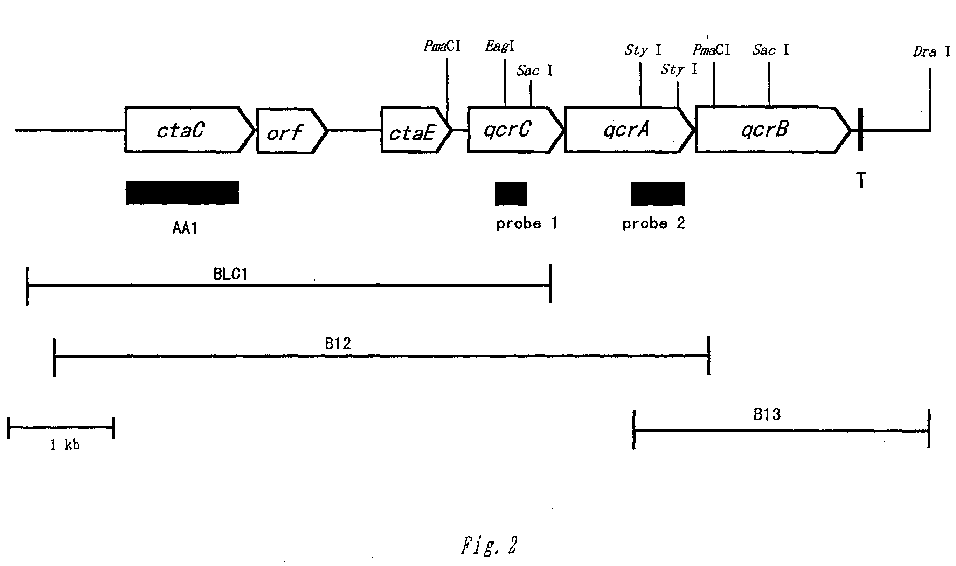

The nucleotide sequence of DNA coding for cytochrome bc1 complex of *Corynebacterium glutamicum* KY9002 strain (ATCC13032) obtained in the manner described above in the working example to be mentioned later is shown in SEQ ID NO: 6. This nucleotide sequence contains six ORFs, three of which (nucleotide numbers 276-1124, 1172-2347 and 2347-3963) are *qcrC, qcrA* and *qcrB,* respectively, and code for the subunits constituting the cytochrome bc1 complex (QcrC, QcrA, QcrB). These genes take an operon structure (Fig. 2). The amino acid sequences encoded by the ORFs are shown in SEQ ID NOS: 7, 8 and 10 in that order. In addition, the amino acid sequence encoded by *qcrC* and *qcrA* is shown in SEQ ID NO: 6 together with the nucleotide sequence. Further, the amino acid sequence encoded by *qcrB* is shown in SEQ ID NO: 9 together with the nucleotide sequence (the same as the nucleotide sequence shown in SEQ ID NO: 6).

The nucleotide sequences of *qcrC, qcrA* and *qcrB* were elucidated by the present invention, and therefore these genes can be directly obtained by preparing primers based on the nucleotide sequence shown in SEQ ID NO: 6, and performing PCR utilizing them and *Corynebacterium glutamlcum* chromosomal DNA as a template.

The DNA of the present invention may be one coding for either one of the aforementioned subunits QcrC, QcrA and QcrB of the cytochrome bc1 complex, or one coding for two or three kinds of them. The cytochrome bc1 complex or a subunit thereof can be produced by introducing the DNA of the present invention into a suitable host cell, and culturing an obtained transformant to express the DNA. For example, the DNA coding for QcrC may be a DNA having the nucleotide sequence consisting of nucleotide numbers 276-1124 in the nucleotide sequence of SEQ ID NO: 6, the DNA coding for QcrA may be a DNA having the nucleotide sequence consisting of nucleotide numbers 1172-2347 in the nucleotide sequence of SEQ ID NO: 6, and the DNA coding for QcrB may be a DNA having the nucleotide sequence consisting of nucleotide numbers 2347-3963 in the nucleotide sequence of SEQ ID NO: 6.

Produced cytochrome bc1 complex or subunits thereof can be collected and purified from culture by a method commonly used for the purification of proteins such as salting out, solvent precipitation, gel filtration chromatography and ion exchange chromatography.

The DNA of the present invention coding for QcrC may be one coding for a polypeptide that has the amino acid sequence of SEQ ID NO: 7 comprising substitution, deletion, insertion or addition of one or several amino acid residues in the amino acid sequence, and can constitute the cytochrome bc1 complex together with QcrA and QcrB.

The DNA of the present invention coding for QcrA may be one coding for a polypeptide that has the amino acid sequence of SEQ ID NO: 8 comprising substitution, deletion, insertion or addition of one or several amino acid residues in the amino acid sequence, and can constitute the cytochrome bc1 complex together with QcrC and QcrB.

The DNA of the present invention coding for QcrB may be one coding for a polypeptide that has the amino acid sequence of SEQ ID NO: 10 comprising substitution, deletion, insertion or addition of one or several amino acid residues in the amino acid sequence, and can constitute the cytochrome bc1 complex together with QcrC and QcrA.

Furthermore, a DNA coding for a cytochrome bc1 complex in which QcrC, QcrA or QcrB, or two or three of them contain a mutation also falls within the scope of the DNA of the present invention.

The number of "several" amino acid residues is preferably 1-40, more preferably 1-10. Alternatively, the number is preferably such a number that the amino acid sequence should show homology of 80% or more, preferably 95% or more, to the amino acid sequence of SEQ ID NO: 7, 8 or 10.

Such a DNA coding for substantially the same protein as QcrC, QcrA or QcrB as described above can be obtained by the site-directed mutagenesis method or mutation treatment in the same manner as those mentioned above for DNA coding for substantially the same protein as cytochrome aa3.

A DNA coding for substantially the same protein as QcrC, QcrA or QcrB can also be obtained by isolating a DNA hybridizable with a DNA having, for example, the nucleotide sequence corresponding to nucleotide numbers of 276-1124, the nucleotide sequence corresponding to nucleotide numbers of 1172-2347 or the nucleotide numbers of 2347-3963 of the nucleotide sequence shown in SEQ ID NO: 6 under the stringent conditions, and coding for a protein having a function of QcrC, QcrA or QcrB from *qcrC, qcrA* or *qcrB* including a mutation or a cell harboring it. The "stringent conditions" has the same meaning as described above.

The host for the expression of the DNA of the present invention include, for example, various kinds of bacteria including coryneform bacteria such as *Escherichia coli, Brevibacterium lactofermentum*, and *Brevibacterium flavum,* eucaryotic cells such as *Saccharomyces cerevisiae* and so forth. In order to introduce the DNA of the present invention into a host such as those mentioned above, the host cell can be transformed with a recombinant vector which is obtained by inserting the DNA of the present invention into a vector selected depending on the kind of the host in which the expression is to be obtained. Those procedures can be performed by using methods of genetic recombination well known to those skilled in the art.

The cytochrome bc1 complex, subunits thereof and DNA coding for them of the present invention are considered to be useful for elucidation of the electron transport system of coryneform bacteria. Further, the DNA can be used for breeding of coryneform bacteria that produce useful substances with good energy efficiency.

### Best Mode for Carrying out the Invention

The present invention will be more specifically explained with reference to the following examples.

### Example 1: Cloning of cytochrome aa3 subunit I gene

### (1) Cloning of cytochrome aa3 subunit I gene of Corynebacterium glutamicum

Primers (uni1: TCATGGTNTGGGYNCAYCAY (SEQ ID NO: 11), uni2r: ATAACRTWRTGRAARTGNGC (SEQ ID NO: 12)) were synthesized based on the amino acid sequence of the region highly conserved in cytochrome c oxidase subunits I of *Bradyrhizobium japonicum, Acetobacter* aceti, *Synechococcus vulcanus* and *Bacillus stearothermophilus.* By using these primers and chromosomal DNA of *Corynebacterium glutamicum* KY9002 strain (ATCC13032) as a template, PCR was performed (95°C for 45 seconds, 52°C for 60 seconds and 62°C for 90 seconds, 35 cycles) to obtain a fragment of 0.3 kb.

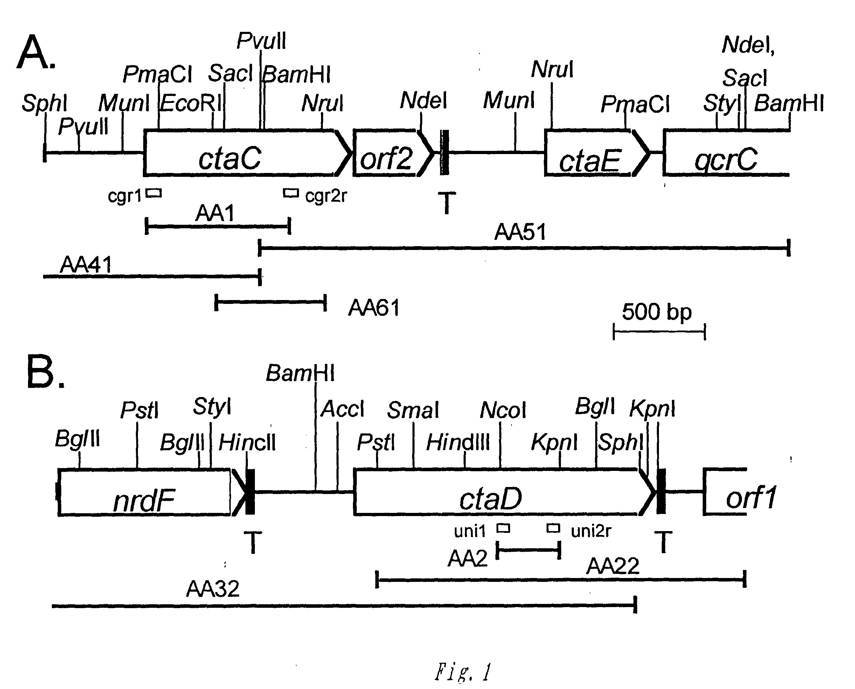

A labeled probe was produced in the same manner as the method described above by using the obtained fragment of 0.3 kb. By using this probe, Southern hybridization was performed for *Corynebacterium glutamicum* chromosomal DNA digested with *Pst*I or *Sph*I. DNA was extracted from the gel at a position corresponding to a position at which a positive signal was obtained. The DNA was ligated to pUC119 digested with *Pst*I or *Sph*I to prepare a recombinant DNA, and *Escherichia coli* was transformed with it. By using the aforementioned probe, colony hybridization was performed for colonies of transformants. Plasmids were prepared from colonies showing positive results for the hybridization to obtain clones AA22 and AA32. The nucleotide sequences of these clones were determined. The nucleotide sequence obtained by ligating the nucleotide sequences of these clones is shown in SEQ ID NO: 1. This nucleotide sequence contained two ORFs (Fig. 1B). As a result of database searching for these ORFs, it was found that the second ORF (nucleotide numbers 1538-3289) was the *ctaD* gene coding for the subunit I of aa3.

### (2) Purification of cytochrome aa3 of Corynebacterium glutamicum

Cultured bacterial cells (wet weight: 120 g) of *Corynebacterium glutamicum* KY9002 strain (ATCC13032) were suspended in 200 ml of a buffer (0.5% NaCl, 10 mM sodium phosphate, pH 7.4), and immediately disrupted by stirring at a high speed by means of a bead beater (Biospec) in the presence of glass beads (D=0.5mm, 350 g). After this disrupted cell suspension was centrifuged at 8,000 x g for 10 minutes to remove undisrupted bacterial cells, the supernatant was centrifuged at 100,000 x g for 1 hour and the resulting precipitates were suspended in a buffer (100 mM NaCl, 50 mM potassium phosphate, pH 6.5) to obtain a membrane preparation.

The aforementioned membrane preparation was suspended in a buffer (5 mg/ml, 2% sodium cholate, 0.5 M NaCl, 10 mM sodium phosphate, pH 7.4), disrupted by sonication, and centrifuged at 100,000 x g for 30 minutes. The resulting precipitates were suspended in a buffer (10 mg/ml, 2% n-octyl-D-glucoside, 0.5 M NaCl, 50 mM potassium phosphate, pH 6.5), and disrupted again by sonication. The disrupted suspension was centrifuged at 100,000 x g for 30 minutes, and the obtained supernatant was dialyzed against a solution of 10 mM sodium phosphate, pH 7.4, and loaded on a DEAE-Toyopearl column (1.4 x 10 cm) so that proteinaceous components should be adsorbed on the column. The proteinaceous components were eluted with an eluent of 1% n-octyl-D-glucoside, 10 mM potassium phosphate, pH 7.4 with increasing NaCl concentration. A fraction showing spectra of heme a and heme a3 in the oxidation-reduction difference spectrum and CO binding reduction-type minus reduction-type difference spectrum was eluted with a condition of 200 mM NaCl as a single peak. This fraction was loaded on a hydroxyapatite eolumn (0.8 x 2 cm) so that the proteinaceous components should be adsorbed on the column and eluted with an eluent of 1% n-octyl-D-glucoside containing sodium phosphate at a gradually increasing concentration. Since the fraction eluted with 200 mM sodium phosphate similarly showed spectra of heme a and heme a3 in the oxidation-reduction difference spectrum and CO binding reduction-type minus reduction-type difference spectrum and TMPD oxidase activity, it was concluded that it was a fraction containing cytochrome aa3 (Table 1).

**Table 1**

| | Total protein | Cytochrome aa3 | | TMPD oxidase activity | | |
|---|---|---|---|---|---|---|
| | | | | Total activity | Specific activity | TN |
| | (mg) | (mmol) | (nmol/ mg) | (unit) | (unit/ mg) | (s-1) |
| Membrane | 662 | 41.0 | 0.06 | 26.7 | 0.04 | 10.8 |
| Cholic acid washing | 583 | 42.7 | 0.07 | 19.4 | 0.033 | 7.51 |
| Octylglucoside extraction | 47.8 | 44.2 | 0.93 | 1.82 | 0.038 | 0.68 |
| DEAE-Toyopearl | 6.19 | 13.2 | 2.13 | 1.51 | 0.242 | 1.91 |
| Hydroxyapatite | 0.34 | 2.8 | 8.09 | 0.110 | 0.296 | 0.61 |

### (3) Sequencing of N-terminus amino acid sequence of cytochrome aa3 subunit II protein

The cytochrome aa3 protein obtained in the manner described above was separated by SDS-PAGE and transferred to a polyvinylidene difluolide membrane. A portion of this membrane at a position considered to correspond to the subunit II was excised and used for amino acid sequencing in Pulse-liquid peptide sequencer Model 477A (Applied Biosystems). The determined amino acid sequence of the N-terminus portion is shown in SEQ ID NO: 13.

### (4) Cloning of cytochrome aa3 subunit II and III genes

A primer crg1 (GGYGAYTTCYTBCGNATGGG, SEQ ID NO: 14) was synthesized based on the amino acid sequence of the N-teminus amino acid sequence of the purified aa3 subunit II. Further, a primer crg2r (GGACCGCASARYTCNGMRCA, SEQ ID NO: 15) was synthesized based on the conserved sequence of CuA binding motif in the oxidase subunits II of *Paracoccus denitrificans, Rhodobacter sphaeroides, Synechococcus vulcanus, Thermus thermophilus, Bacillus subtilis* and *Bacillus stearothermophilus.* By using these crg1 and crg2r, and chromosomal DNA of *Corynebacterium glutamicum* KY9002 strain (ATCC13032) as a template, PCR was performed (95°C for 60 seconds, 52°C for 60 seconds, 68°C for 60 seconds, 35 cycles) to obtain a fragment of 0.8 kb (AA1). The chromosomal DNA of *Corynebacterium glutamicum* was prepared by the method of Saito and Miura *(Biochem. Biophys. Acta., 72,* 619 (1963)).

The obtained fragment of 0.8 kb was labeled by using DIG DNA Labeling Kit (Bohringer Mannheim), and used as a probe for screening of a chromosomal DNA library. The used chromosomal DNA library of *Corynebacterium glutamicum* was obtained by partially digesting the chromosomal DNA with a restriction enzyme *Sau*3AI, ligating the obtained DNA fragments to pUC119 digested with a restriction enzyme *Bam*HI to produce recombinant DNA, and transforming *Escherichia coli* with the recombinant DNA.

Colony hybridization (47-50°C, 5 x SSC, 0.5% blocking reagent (Bohringer Mannheim), 0.1% sodium lauroyl sarcosinate, 0.02% SDS) was performed for the colonies of transformants by using the aforementioned probe labeled with DIG. The probe was detected by using DIG Detection Kit (Bohringer Mannheim), which utilized anti-DIG antibodies labeled with alkaline phosphatase. Plasmids were prepared from colonies that showed positive results for the hybridization to obtain clones AA41, AA51 and AA61. The nucleotide sequences of these clones were determined. It was found that these nucleotide sequences overlapped with on another and contained the *ctaC* gene coding for the subunit II and the *ctaE* gene coding for the subunit III of aa3 as a whole (Fig. 1A). A nucleotide sequence obtained by ligating the nucleotide sequences of the aforementioned three clones is shown in SEQ ID NO: 3. This nucleotide sequence contained three open reading frames (ORF). As a result of database searching for these ORFs, it was found that the first ORF (nucleotide numbers 604-1680) was the *ctaC* gene coding for the subunit II, and the third OFR (nucleotide numbers 2715-3329) was the *ctaE* gene coding for the subunit III. The amino acid sequences therefor are shown in SEQ ID NOS: 4 and 5, respectively.

The results of homology searching of databases for each amino acid sequence are shown in Table 2. The sequence analysis was performed by using GENETYX Homology Version 2.2.2 (Software Development Co., Ltd.). The homology analysis was performed according to the method of Lipman and Peason (Science, 227, 1435-1441, 1985).

**Table 2**

| Gene | Homology (amino acid sequence level) | |
|---|---|---|
| *ctaD* | *Mycobacterium tuberculosis Bacillus stearothermohilus* | *ctaD* product (70%) Cytochrome oxidase subunit I (47%) |
| *ctaC* | *Mycobacterium tuberculosis Streptomyces coelicolor* | *ctaC* product (50%) Protein estimated to be cytochrome oxidase subunit II (32%) |
| *ctaE* | *Mycobacterium* tuberculosis *Streptomyces coelicolor* | *ctaE* product (60%) Protein estimated to be cytochrome oxidase subunit III (57%) |

### Example 2: Cloning of cytochrome bc1 complex gene

A sequence estimated to be a part of ORF was found in the sequence of SEQ ID NO: 3 obtained in Example 1 at a position downstream from the gene coding for cytochrome aa3 subunit III, *ctaE,* on the 3' end side. As a result of database searching for the amino acid sequence that may be encoded by this sequence, the amino acid sequence showed homology to an amino acid sequence of known cytochrome c1 subunit.

The partial sequence of about 800 bp used for the cloning of *ctaC* in Example 1 was obtained by PCR, labeled by using DIG DNA Labeling Kit (Bohringer Mannheim), and designated as Probe AA1. By using this probe AA1, a chromosomal DNA library was screened through colony hybridization. The used chromosomal DNA library was obtained by partially digesting the chromosomal DNA of *Corynebacterium glutamicum* with a restriction enzyme *Sau*3AI, ligating the obtained DNA fragments to pUC18 digested with a restriction enzyme *Bam*HI to produce recombinant DNA, and transforming *Escherichia coli* with the recombinant DNA. The probe was detected by using DIG Detection Kit (Bohringer Mannheim), which utilized anti-DIG antibodies labeled with alkaline phosphatase. Plasmids were prepared from colonies that showed positive results for the hybridization to obtain clone BLC1.

The nucleotide sequence of the aforementioned clone pBLC1 was determined, and a fragment of 154 bp was newly excised from that sequence with restriction enzymes *Eag*I and *Sac*I and designated as Probe 1. This Probe 1 was labeled in the same manner as described above, and used to screen again the chromosomal DNA library. The nucleotide sequence of clone B12 obtained by the above procedure was determined, and a fragment of about 500 bp excised from the sequence with a restriction enzyme *Sty*I was used to produce a probe (Probe 2). This Probe 2 was labeled in the manner described above, and used to screen a chromosomal DNA library through colony hybridization. The chromosomal DNA library used in this screening was obtained by fully digesting the chromosomal DNA of *Corynebacterium glutamicum* with restriction enzymes *Bgl*II and *Sma*I, collecting DNA fragments of about 2.8 kb, ligating the fragment to pUC118 digested with restriction enzymes SmaI and *Bam*HI to produce recombinant DNA, and transforming *Escherichia coli* with the recombinant DNA. This procedure provided a clone B13, and its nucleotide sequence was determined. A nucleotide sequence obtained by ligating BLC1, B12 and B13 is shown in SEQ ID NO: 6. This nucleotide sequence contained six ORFs, three of which (nucleotide numbers 276-1124, 1172-2347 and 2347-3963) were in an operon structure (Fig. 2). Databases were searched for these OFRs, and they were designated as *qcrC, qcrA* and *qcrB,* respectively. The amino acid sequences encoded by these ORFs are shown in SEQ ID NOS: 7, 8 and 10, respectively. In addition, the amino acid sequence encoded by *qcrC* and *qcrA* is shown in SEQ ID NO: 6 together with the nucleotide sequence. Further, the amino acid sequence encoded by *qcrB* is shown in SEQ ID NO: 9 together with the nucleotide sequence (the same the nucleotide sequence shown in SEQ ID NO: 6).

The *qcrC* gene product was characterized by containing two of heme c binding motifs, and showed 34% or more of homology on amino acid level to homologous proteins of high G+C content gram positive bacteria such as *Mycobacterium tuberculosis* and *Streptomyces coelicolor.* In particular, it showed a high homology of 54% to the homologous protein of *M. tuberculosis.* It was concluded that this *qcrC* was the gene coding for the cytochrome c1 subunit of cytochrome bc1 complex.

Further, the *qcrA* gene product contained a Rieske iron-sulfur motif, although the conservation degree was low, and showed the highest homology to the Rieske iron-sulfur protein of *Thermus thermophilus* among the high G+C content gram positive bacteria. It was concluded that this *qcrA* was the gene coding for Rieske iron-sulfur protein of the cytochrome bc1 complex.

The *qcrB* gene product showed homology to cytochrome b of high G+C content gram positive bacterium, *T. thermophilus,* and cytochrome b6 of *Bacillus stearothermopholus,* and a His residue that is a heme bonding site and peripheral sequences thereof were also highly conserved in it. It is expected that these gene products are functioning by forming a complex with cytochrome c1, iron-sulfur protein or cytochrome b, as is already known for mitochondria of eukaryotes and *Proteobacteria.* It was concluded that this *qcrB* was the gene coding for the cytochrome b subunit of cytochrome bc1 complex.

The sequence analysis was performed by using GENETYX Homology Version 2.2.2 (Software Development Co., Ltd.). The homology analysis was performed according to the method of Lipman and Peason (Science, 227, 1435-1441, 1985).

**Table 3**

| Gene | Homology (amino acid sequence level) |
|---|---|
| *qcrC* | *qcrC* product of high G+C content gram positive bacteria (34% or more) *qcrC* product of M. *tuberculosis* (54%) |
| *qcrA* | *T. thermophilus* Rieske iron-sulfur protein (45%) |
| *qcrB* | *M. tuberculosis* cytochrome b (61%) *B. stearothermopholus* cytochrome b6 (31%) |

### Example 3: Purification of heme c containing protein of Corynebacterium glutamicum

### (1) Purification of heme c containing protein

In cytochrome c, heme c is covalently bonded to a cysteine residue of a protein portion, and therefore the heme c is still bonded to it even after the protein is denatured. The heme iron has peroxidase activity. Therefore, among polypeptides obtained by denaturing cytochrome c, presence of those having a covalently bonded heme can be clarified. Denatured cytochrome c was subjected to SDS-polyacrylamide gel electrophoresis, and peroxidase activity was detected. As a result, it was found that only the polypeptide having a molecular weight of about 28 kDa showed the peroxidase activity, and had c type heme. Based on this, it is considered that *Corynebacterium glutamicum* has one kind of protein containing heme c. Moreover, it is considered that, if this protein is purified and its amino acid sequence is determined, the subunit of cytochrome bc1 complex can be identified.

Cultured cells of *Corynebacterium glutamicum* KY9002 strain (ATCC13032) were collected by centrifugation at 10,000 x g for 10 minutes, washed with a buffer (20 mM potassium phosphate, pH 7.5), and collected again by centrifugation at 10,000 x g for 10 minutes. This washing procedure was repeated again, and the obtained cells were suspended in a buffer (20 mM potassium phosphate, pH 7.5), added with lysozyme (Sigma) at a final concentration of 0.5 mg/ml, left at room temperature for 30 minutes, and disrupted by sonication.

This disrupted cell suspension was centrifuged at 10,000 x g for 20 minutes to remove undisrupted cells, and the supernatant was centrifuged at 150,000 x g for 90 minutes to obtain precipitates, which were suspended in a buffer (20 mM potassium phosphate, pH 7.5) for washing. The suspension was centrifuged at 150,000 x g for 90 minutes, and the obtained precipitates were suspended in 200 ml of buffer (1% sodium cholate, 0.5% sodium deoxycholate, 0.1 M NaCl, 10 mM sodium phosphate, pH 6.8) at a protein concentration of 10 mg/ml for washing, and the suspension was centrifuged at 100,000 x g for 45 minutes to precipitate membrane proteins.

The membrane proteins obtained as described above were dissolved in a buffer (1% decylglucoside, 0.05 M potassium phosphate, pH 6.5) by mild sonication, and centrifuged at 100,000 x g for 30 minutes. The obtained supernatant was dialyzed against 20 mM Tris-HCl, pH 7.2 for 3 hours. The dialyzed supernatant was loaded on a DEAE-Toyopearl column (1.6 x 8.0 cm) equilibrated with a buffer (1% decylglucoside, 20 mM Tris-HCl, pH 7.2). The proteins adsorbed on the column were eluted with a buffer as eluent (1% n-octyl-D-glucoside, 20 mM Tris-HCl, pH 7.2) containing NaCl with increasing concentration. A fraction in red color containing heme c and exulted at 60-80 mM of NaCl concentration was collected, and loaded on a hydroxyapatite column (0.5 x 1.5 cm) so that the proteinaceous components should be adsorbed on the column. The column was washed with a buffer (1% n-octyl-D-glucoside, 20 mM Tris-HCl, pH 7.2), and then the proteinaceous components were eluted with a buffer (1% decylglucoside, 5 mM sodium phosphate, pH 6.8). The obtained fraction was concentrated to about 0.1 mL by loading it to Centricon 30 (Amersham), and subjected to gel filtration by loading it at a flow rate of 0.8 mL/min to a Toso G3000SW column (Tosoh) equilibrated with a buffer (0.05% decylglucoside, 0.1 M NaCl, 10 mM Tris-HCl, pH 7.2) by HPLC to obtain a fraction containing a protein containing heme c (Table 4).

**Table 4**

| | Total protein | Heme c | |
|---|---|---|---|
| | (mg) | (mmol) | (nmol/mg) |
| Membrane | 169 | 120.0 | 0.07 |
| Cholic acid washing | 166 | 99.0 | 0.06 |
| Octylglucoside extraction | 4.5 | 33.0 | 7.3 |
| DEAE-Toyopearl | 0.45 | 12.7 | 29 |
| Hydroxyapatite | 0.34 | 9.3 | 62 |

### (2) Determination of N-terminus amino acid sequence of heme c containing protein

The fraction containing heme c containing protein obtained as described above was separated by SDS-PAGE and transferred to a polyvinylidene difluoride membrane. A portion of this membrane at a position corresponding to cc1 was excised, and the proteins were degraded composed by a previously reported method (Kuge, S. *et al., Biochem. Mol. Biol. Int., 38,* 181-188 (1996)) using *Staphylococcus aureus* V8 protease. The degradation product was subjected to amino acid sequence determination using Pulse-liquid peptide sequencer Model 477A (Applied Biosystems). As a result, two kinds of partial amino acid sequences, QAERKAPRITEAQVLA (SEQ ID NO: 16) and LRGENYDGQITSADVARGGDLFRL (SEQ ID NO: 17), were obtained. These partial amino acid sequences were substantially the same as the amino acid sequence obtained from the aforementioned cloned *qcrC* gene.

<Explanation of Sequence Listing>
SEQ ID NO: 1: nucleotide sequence of ctaD and amino acid
sequence of CtaD,
SEQ ID NO: 2: amino acid sequence of CtaD,
SEQ ID NO: 3: nucleotide sequences of ctaC and ctaE and
amino acid sequences of Cta C and CtaE
SEQ ID NO: 4: amino acid sequence of CtaC
SEQ ID NO: 5: amino acid sequence of CtaE
SEQ ID NO: 6: nucleotide sequence of qcrCAB and amino
acid sequences of QcrC and QcrA
SEQ ID NO: 7: amino acid sequence of QcrC
SEQ ID NO: 8: amino acid sequence of QcrA
SEQ ID NO: 9: nucleotide sequence of qcrCAB and amino
acid sequence of QcrB
SEQ ID NO: 10: amino acid sequence of QcrB
SEQ ID NO: 11: primer uni1
SEQ ID NO: 12: primer uni1r
SEQ ID NO: 13: N-terminus amino acid sequence of
cytochrome aa3 subunit II protein
SEQ ID NO: 14: primer crg1
SEQ ID NO: 15: primer crg2r
SEQ ID NO: 16: partial amino acid sequence of cytochrome
cc1 protein
SEQ ID NO: 17: partial amino acid sequence of cytochrome
cc1 protein

## Claims

1. A polypeptide defined in the following (A1) or (A2):
(A1) a polypeptide that has the amino acid sequence of SEQ ID NO: 2,
(A2) a polypeptide that has the amino acid sequence of SEQ ID NO: 2 comprising substitution, deletion, insertion or addition of one or several amino acid residues in the amino acid sequence, and can constitute cytochrome aa3 together with a cytochrome aa3 subunit II having the amino acid sequence of SEQ ID NO: 4 and cytochrome aa3 subunit III having the amino acid sequence of SEQ ID NO: 5.

2. A polypeptide defined in the following (B1) or (B2):
(B1) a polypeptide that has the amino acid sequence of SEQ ID NO: 4,
(B2) a polypeptide that has the amino acid sequence of SEQ ID NO: 4 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit III having the amino acid sequence of SEQ ID NO: 5.

3. A polypeptide defined in the following (C1) or (C2):
(C1) a polypeptide that has the amino acid sequence of SEQ ID NO: 5,
(C2) a polypeptide that has the amino acid sequence of SEQ ID NO: 5 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit II having the amino acid sequence of SEQ ID NO: 4.

4. A cytochrome aa3 consisting of the polypeptides according to Claims 1, 2 and 3.

5. A polypeptide defined in the following (D1) or (D2):
(D1) a polypeptide that has the amino acid sequence of SEQ ID NO: 7,
(D2) a polypeptide that has the amino acid sequence of SEQ ID NO: 7 comprising substitution, deletion, insertion or addition of one or several amino acid residues in the amino acid sequence, and can constitute a cytochrome bc1 complex together with QcrA having the amino acid sequence of SEQ ID NO: 8 and QcrB having the amino acid sequence of SEQ ID NO: 10.

6. A polypeptide defined in the following (E1) or (E2):
(E1) a polypeptide that has the amino acid sequence of SEQ ID NO: 8,
(E2) a polypeptide that has the amino acid sequence of SEQ ID NO: 8 comprising substitution, deletion, insertion or addition of one or several amino acid residues in the amino acid sequence, and can constitute a cytochrome bc1 complex together with QcrC having the amino acid sequence of SEQ ID NO: 7 and QcrB having the amino acid sequence of SEQ ID NO: 10.

7. A polypeptide defined in the following (F1) or (F2):
(F1) a polypeptide that has the amino acid sequence of SEQ ID NO: 10,
(F2) a polypeptide that has the amino acid sequence of SEQ ID NO: 10 comprising substitution, deletion, insertion or addition of one or several amino acid residues in the amino acid sequence, and can constitute a cytochrome bc1 complex together with QcrC having the amino acid sequence of SEQ ID NO: 7 and QcrA having the amino acid sequence of SEQ ID NO: 8.

8. A cytochrome bc1 complex consisting of the polypeptides according to Claims 5, 6 and 7.

9. A DNA coding for a polypeptide defined in the following (A1) or (A2):
(A1) a polypeptide that has the amino acid sequence of SEQ ID NO: 2,
(A2) a polypeptide that has the amino acid sequence of SEQ ID NO: 2 comprising substitution, deletion, insertion or addition of one or several amino acid residues in the amino acid sequence, and can constitute cytochrome aa3 together with a cytochrome aa3 subunit II having the amino acid sequence of SEQ ID NO: 4 and cytochrome aa3 subunit III having the amino acid sequence of SEQ ID NO: 5.

10. A DNA coding for a polypeptide defined in the following (B1) or (B2):
(B1) a polypeptide that has the amino acid sequence of SEQ ID NO: 4,
(B2) a polypeptide that has the amino acid sequence of SEQ ID NO: 4 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit III having the amino acid sequence of SEQ ID NO: 5.

11. A DNA coding for a polypeptide defined in the following (C1) or (C2):
(C1) a polypeptide that has the amino acid sequence of SEQ ID NO: 5,
(C2) a polypeptide that has the amino acid sequence of SEQ ID NO: 5 comprising substitution, deletion, insertion or addition of one or several amino acid residues in the amino acid sequence, and can constitute cytochrome aa3 together with a cytochrome aa3 subunit I having the amino acid sequence of SEQ ID NO: 2 and cytochrome aa3 subunit II having the aminoacid sequence of SEQ ID NO: 4.

12. A DNA coding for a polypeptide defined in the following (D1) or (D2):
(D1) a polypeptide that has the amino acid sequence of SEQ ID NO: 7,
(D2) a polypeptide that has the amino acid sequence of SEQ ID NO: 7 comprising substitution, deletion, insertion or addition of one or several amino acid residues in the amino acid sequence, and can constitute a cytochrome bc1 complex together with QcrA having the amino acid sequence of SEQ ID NO: 8 and QcrB having the amino acid sequence of SEQ ID NO: 10.

13. A DNA coding for a polypeptide defined in the following (E1) or (E2):
(E1) a polypeptide that has the amino acid sequence of SEQ ID NO: 8,
(E2) a polypeptide that has the amino acid sequence of SEQ ID NO: 8 comprising substitution, deletion, insertion or addition of one or several amino acid residues in the amino acid sequence, and can constitute a cytochrome bc1 complex together with QcrC having the amino acid sequence of SEQ ID NO: 7 and QcrB having the amino acid sequence of SEQ ID NO: 10.

14. A DNA coding for a polypeptide defined in the following (F1) or (F2):
(F1) a polypeptide that has the amino acid sequence of SEQ ID NO: 10,
(F2) a polypeptide that has the amino acid sequence of SEQ ID NO: 10 comprising substitution, deletion, insertion or addition of one or several amino acid residues in the amino acid sequence, and can constitute a cytochrome bc1 complex together with QcrC having the amino acid sequence of SEQ ID NO: 7 and QcrA having the amino acid sequence of SEQ ID NO: 8.


SEQUENCE LISTING

<110> Ajinomoto Co., Inc

<120> Respiratory Chain Enzyme Genes of Coryneform Bacteria

<130> EPA-53732

<160> 17

<170> PatentIn Ver. 2.0

<210> 1
<211> 3804
<212> DNA
<213> Corynebacterium glutamicum

<220>
<221> CDS
<222> (1538)..(3289)

<400> 1
agagcgcacc taatggctgc tgattctgat ctcagtgttc acgatgctta cttaaaggag 60
catgttgcac ctgtaaaggc gatcaactgg aactccatcc cagattccaa agatcttgaa 120
gtctgggatc gtctgaccgg taacttctgg ctcccagaaa aggtcccagt atccaacgac 180
atcaagagct ggggaaccct caacgaggtt gaaaaagccg caaccatgcg cgtgttcacc 240
ggacttaccc tgctggacac cattcagggc actgtcggcg caatctccct gcttccagac 300
gcagattcac tgcacgaaga agcggtgcta accaacattg cgttcatgga atccgtgcac 360
gcaaagagtt actccaacat cttcatgact ctggcctcca ccgcggaaat caacgatgcg 420
ttccgttggt ctgaggaaaa tgaaaacctg cagcgcaagg caaagatcat cctgtcttac 480
tatgagggcg atgatccact aaagcgcaag atcgcctccg tgatcctgga gtccttcctg 540
ttctactccg gcttctacct cccaatgtat tggtccagcc actccaagct gaccaacacc 600
gccgacgtga tccgcctgat catccgcgat gaggcagtgc acggctacta cattggctac 660

aagtatcaaa aggctgtcgc gaaggagact ccagagcgtc aggaagagct gaaggagtac 720
accttcgatc tgctctacga tctttacgat aacgaaactc agtactccga agatctctac 780
gacgatcttg gatggaccga ggatgttaag cgattccttc gctacaacgc caacaaggcc 840
ctcaacaacc ttggctacga aggactcttc ccagcggatg aaaccaaggt gtccccaaac 900
atcttgtctg cgctgtcacc aaacgctgat gagaaccacg acttcttctc cggctccggt 960
tcctcttacg ttattggtaa ggcagaaaac accgaggatg atgactggga cttctaactt 1020
ttaaaaagct gaagcgctct acggctgtag ttaactgcaa ccgttagagc gtttttcgct 1080
ttctggtggg ggcttaaggt gcgggttttt ccgaagcgca tatcggggta ggggagcgcc 1140
aggcgccccg tggacccctc ggcggcacat cacgctttag aagaaaacgc ccctggaatg 1200
ggcgtctcaa ccattcgatt gaaccccggc ggggggaatt gtgaaatctg tggcaggggt 1260
taaccgtggg ggtagggctt cctggcgaaa tgtccgtcaa attgtgaacc ccttcacacc 1320
tttggttaaa agtcactgcc cacaagtgac tgaacctggc agcgacctca tgaattgttt 1380
gaaaaacatt ttttttgggc atgaaaaggg gatacagtta gctgcatacc ggcctttttg 1440
ggttggcatc ggatcctgcc tgtggcctaa gatcaggcag tgttgttaaa ggacgatcgg 1500
taatccgaat ggatcgtccc gtagtcagga ggaacct atg acc gct gtg gcg cct 1555
Met Thr Ala Val Ala Pro
1               5
agg gtc gac ggg cac gtc gcc cct cag agg ccc gag ccg aca ggc cat 1603
Arg Val Asp Gly His Val Ala Pro Gln Arg Pro Glu Pro Thr Gly His
            10                  15                  20
gca cgc aag ggc agc aaa gca tgg tta atg atg acc acc acc gac cac 1651
Ala Arg Lys Gly Ser Lys Ala Trp Leu Met Met Thr Thr Thr Asp His
        25                  30                  35
aag cag ctg ggc att atg tac atc att atg tcc ttc agc ttc ttc ttc 1699
Lys Gln Leu Gly Ile Met Tyr Ile Ile Met Ser Phe Ser Phe Phe Phe
    40                  45                  50
ctc ggt ggc ttg atg gcc ctg ctt atc cga gcg gag ctt ttc acc cct 1747
Leu Gly Gly Leu Met Ala Leu Leu Ile Arg Ala Glu Leu Phe Thr Pro
55                  60                  65                  70
ggt ctg cag ttc ctg tct aat gag cag ttc aac cag ctg ttc acc atg 1795
Gly Leu Gln Phe Leu Ser Asn Glu Gln Phe Asn Gln Leu Phe Thr Met
                75                  80                  85
cac gga act gtc atg ctg ctg ctg tac gga act cca att gtt tgg ggt 1843
His Gly Thr Val Met Leu Leu Leu Tyr Gly Thr Pro Ile Val Trp Gly
            90                  95                  100
ttt gct aac tac gtc ctg cca ctt cag atc ggt gcg cct gac gta gct 1891
Phe Ala Asn Tyr Val Leu Pro Leu Gln Ile Gly Ala Pro Asp Val Ala
        105                 110                 115
ttc cca cgt ttg aat gct ttc ggc ttc tgg atc acc acc gtc ggt ggt 1939
Phe Pro Arg Leu Asn Ala Phe Gly Phe Trp Ile Thr Thr Val Gly Gly
    120                 125                 130

gtc gcg atg ctg acc ggc ttc ctg acc ccg ggt ggt gct gcc gac ttc    1987
Val Ala Met Leu Thr Gly Phe Leu Thr Pro Gly Gly Ala Ala Asp Phe
135                 140                 145                 150
ggt tgg acc atg tac tcc cca ctg tct gac gca att cac tcc cca ggc    2035
Gly Trp Thr Met Tyr Ser Pro Leu Ser Asp Ala Ile His Ser Pro Gly
                155                 160                 165
ctt ggc tct gac atg tgg att gtc ggt gtc ggt gca act ggt att ggc    2083
Leu Gly Ser Asp Met Trp Ile Val Gly Val Gly Ala Thr Gly Ile Gly
            170                 175                 180
tcc gtt gct tcc gca att aac atg ctc acc acc atc ctc tgc ctc cgc    2131
Ser Val Ala Ser Ala Ile Asn Met Leu Thr Thr Ile Leu Cys Leu Arg
        185                 190                 195
gca cct ggt atg acc atg ttc cgt atg cct att ttc acc tgg aat atc    2179
Ala Pro Gly Met Thr Met Phe Arg Met Pro Ile Phe Thr Trp Asn Ile
    200                 205                 210
ttc gtt gtt tcc gtt ctt gct ctg ctg atc ttc cca ctg ctg ctc gct    2227
Phe Val Val Ser Val Leu Ala Leu Leu Ile Phe Pro Leu Leu Leu Ala
215                 220                 225                 230
gct gca ctg ggt gtt ctg tat gac cgc aag ctt ggt gga cac ctg tac    2275
Ala Ala Leu Gly Val Leu Tyr Asp Arg Lys Leu Gly Gly His Leu Tyr
                235                 240                 245
gat cca gct aac ggc ggc tcc ctc ctg tgg cag cac ctg ttc tgg ttc    2323
Asp Pro Ala Asn Gly Gly Ser Leu Leu Trp Gln His Leu Phe Trp Phe
            250                 255                 260
ttc gga cac cct gag gtt tac gtt ctg gcg ctg ccg ttc ttc ggc att    2371
Phe Gly His Pro Glu Val Tyr Val Leu Ala Leu Pro Phe Phe Gly Ile
        265                 270                 275
gtt tct gag atc att cct gtg ttc tcc cgt aag cca atg ttc ggt tac    2419
Val Ser Glu Ile Ile Pro Val Phe Ser Arg Lys Pro Met Phe Gly Tyr
    280                 285                 290
gtc ggc ctg atc ttc gca acc ttg tcc att ggt gca ctg tcc atg gct    2467
Val Gly Leu Ile Phe Ala Thr Leu Ser Ile Gly Ala Leu Ser Met Ala
295                 300                 305                 310
gtg tgg gct cac cac atg ttc gtt act ggc gca gtt ttg ctt ccg ttc    2515
Val Trp Ala His His Met Phe Val Thr Gly Ala Val Leu Leu Pro Phe
                315                 320                 325
ttc tcc ttc atg acg ttc ctg att tcg gtt cct acc ggc gtt aag ttc    2563
Phe Ser Phe Met Thr Phe Leu Ile Ser Val Pro Thr Gly Val Lys Phe
            330                 335                 340
ttc aac tgg gtt gga acc atg tgg aag ggt cac atc act tgg gaa acc    2611
Phe Asn Trp Val Gly Thr Met Trp Lys Gly His Ile Thr Trp Glu Thr

        345                 350                 355
cca atg atc tgg tct gtt ggc ttc atg gct acc ttc ctc ttc ggt ggt 2659
Pro Met Ile Trp Ser Val Gly Phe Met Ala Thr Phe Leu Phe Gly Gly
    360                 365                 370
ctg acc ggc att atg ctg gcg tcc cca cca ctg gac ttc cac ttg gct 2707
Leu Thr Gly Ile Met Leu Ala Ser Pro Pro Leu Asp Phe His Leu Ala
375                 380                 385                 390
gac tcc tac ttc ctg atc gcg cac ttc cac tac acc ctc ttc ggt acc 2755
Asp Ser Tyr Phe Leu Ile Ala His Phe His Tyr Thr Leu Phe Gly Thr
                395                 400                 405
gtg gtg ttc gca tcg tgt gca ggc gtt tac ttc tgg ttc ccg aag atg 2803
Val Val Phe Ala Ser Cys Ala Gly Val Tyr Phe Trp Phe Pro Lys Met
            410                 415                 420
act ggc cgc atg atg gac gag cgt ctt ggc aag atc cac ttc tgg ttg 2851
Thr Gly Arg Met Met Asp Glu Arg Leu Gly Lys Ile His Phe Trp Leu
        425                 430                 435
acc ttc gtc ggt ttc cac gga acc ttc ctc atc cag cac tgg gtg ggc 2899
Thr Phe Val Gly Phe His Gly Thr Phe Leu Ile Gln His Trp Val Gly
    440                 445                 450
aac atg ggt atg cca cgt cgt tac gct gac tac ctg gat tct gat ggt 2947
Asn Met Gly Met Pro Arg Arg Tyr Ala Asp Tyr Leu Asp Ser Asp Gly
455                 460                 465                 470
ttc acc atc tac aac cag atc tcc acc gtg ttc tcc ttc ctg ctt ggc 2995
Phe Thr Ile Tyr Asn Gln Ile Ser Thr Val Phe Ser Phe Leu Leu Gly
                475                 480                 485
ctg tct gtc att cca ttc atc tgg aac gtc ttc aag tcc tgg cgc tac 3043
Leu Ser Val Ile Pro Phe Ile Trp Asn Val Phe Lys Ser Trp Arg Tyr
            490                 495                 500
ggt gag ctc gtt acc gtt gat gat cct tgg ggt tac ggc aac tcc ctg 3091
Gly Glu Leu Val Thr Val Asp Asp Pro Trp Gly Tyr Gly Asn Ser Leu
        505                 510                 515
gag tgg gca acc tcc tgc cct cct cct cgc cac aac ttc gca tcc ttg 3139
Glu Trp Ala Thr Ser Cys Pro Pro Pro Arg His Asn Phe Ala Ser Leu
    520                 525                 530
cct cgt atc cgc tcc gag cgc cct gcg ttc gag ctg cac tac ccg cac 3187
Pro Arg Ile Arg Ser Glu Arg Pro Ala Phe Glu Leu His Tyr Pro His
535                 540                 545                 550
atg att gaa agc atg cgc gca gag gca cac act gga cat cac gat gat 3235
Met Ile Glu Ser Met Arg Ala Glu Ala His Thr Gly His His Asp Asp
                555                 560                 565
att aat gct cca gaa ttg ggt acc gcc cca gcc ctt gca tct gac tcc 3283

Ile Asn Ala Pro Glu Leu Gly Thr Ala Pro Ala Leu Ala Ser Asp Ser
            570                 575                 580
agc cgc taaaagcgtc tgatttaagt cggtacctga ctaaataagc accagcccca 3339
Ser Arg
gcagagataa tctgccgggg ctggcgtttt catattccga cttggggcac cctgaataca 3399
tctcacccaa ttccccataa ctagacaatt gcccagcaac gactgataag tctccaatgt 3459
cgtgttccgc gctcagacat gagacaattg ttgccgtgac tgaactcatc cagaatgaat 3519
cccaagaaat cgctgagctg gaagccggcc agcaggttgc attgcgtgaa ggttatcttc 3579
ctgcggtgat cacagtgagc ggtaaagacc gcccaggtgt gactgccgcg ttctttaggg 3639
tcttgtccgc taatcaggtt caggtcttgg acgttgagca gtcaatgttc cgtggctttt 3699
tgaacttggc ggcgtttgtg ggtatcgcac ctgagcgtgt cgagaccgtc accacaggcc 3759
tgactgacac cctcaaggtg catggacagt ccgtggtggt ggagc 3804

<210> 2
<211> 584
<212> PRT
<213> Corynebacterium glutamicum

<400> 2
Met Thr Ala Val Ala Pro Arg Val Asp Gly His Val Ala Pro Gln Arg
1               5                   10                  15
Pro Glu Pro Thr Gly His Ala Arg Lys Gly Ser Lys Ala Trp Leu Met
            20                  25                  30
Met Thr Thr Thr Asp His Lys Gln Leu Gly Ile Met Tyr Ile Ile Met
        35                  40                  45
Ser Phe Ser Phe Phe Phe Leu Gly Gly Leu Met Ala Leu Leu Ile Arg
    50                  55                  60
Ala Glu Leu Phe Thr Pro Gly Leu Gln Phe Leu Ser Asn Glu Gln Phe
65                  70                  75                  80
Asn Gln Leu Phe Thr Met His Gly Thr Val Met Leu Leu Leu Tyr Gly
                85                  90                  95
Thr Pro Ile Val Trp Gly Phe Ala Asn Tyr Val Leu Pro Leu Gln Ile
            100                 105                 110
Gly Ala Pro Asp Val Ala Phe Pro Arg Leu Asn Ala Phe Gly Phe Trp
        115                 120                 125
Ile Thr Thr Val Gly Gly Val Ala Met Leu Thr Gly Phe Leu Thr Pro
    130                 135                 140
Gly Gly Ala Ala Asp Phe Gly Trp Thr Met Tyr Ser Pro Leu Ser Asp
145                 150                 155                 160
Ala Ile His Ser Pro Gly Leu Gly Ser Asp Met Trp Ile Val Gly Val
                165                 170                 175

Gly Ala Thr Gly Ile Gly Ser Val Ala Ser Ala Ile Asn Met Leu Thr
            180                 185                 190
Thr Ile Leu Cys Leu Arg Ala Pro Gly Met Thr Met Phe Arg Met Pro
        195                 200                 205
Ile Phe Thr Trp Asn Ile Phe Val Val Ser Val Leu Ala Leu Leu Ile
    210                 215                 220
Phe Pro Leu Leu Leu Ala Ala Ala Leu Gly Val Leu Tyr Asp Arg Lys
225                 230                 235                 240
Leu Gly Gly His Leu Tyr Asp Pro Ala Asn Gly Gly Ser Leu Leu Trp
                245                 250                 255
Gln His Leu Phe Trp Phe Phe Gly His Pro Glu Val Tyr Val Leu Ala
            260                 265                 270
Leu Pro Phe Phe Gly Ile Val Ser Glu Ile Ile Pro Val Phe Ser Arg
        275                 280                 285
Lys Pro Met Phe Gly Tyr Val Gly Leu Ile Phe Ala Thr Leu Ser Ile
    290                 295                 300
Gly Ala Leu Ser Met Ala Val Trp Ala His His Met Phe Val Thr Gly
305                 310                 315                 320
Ala Val Leu Leu Pro Phe Phe Ser Phe Met Thr Phe Leu Ile Ser Val
                325                 330                 335
Pro Thr Gly Val Lys Phe Phe Asn Trp Val Gly Thr Met Trp Lys Gly
            340                 345                 350
His Ile Thr Trp Glu Thr Pro Met Ile Trp Ser Val Gly Phe Met Ala
        355                 360                 365
Thr Phe Leu Phe Gly Gly Leu Thr Gly Ile Met Leu Ala Ser Pro Pro
    370                 375                 380
Leu Asp Phe His Leu Ala Asp Ser Tyr Phe Leu Ile Ala His Phe His
385                 390                 395                 400
Tyr Thr Leu Phe Gly Thr Val Val Phe Ala Ser Cys Ala Gly Val Tyr
                405                 410                 415
Phe Trp Phe Pro Lys Met Thr Gly Arg Met Met Asp Glu Arg Leu Gly
            420                 425                 430
Lys Ile His Phe Trp Leu Thr Phe Val Gly Phe His Gly Thr Phe Leu
        435                 440                 445
Ile Gln His Trp Val Gly Asn Met Gly Met Pro Arg Arg Tyr Ala Asp
    450                 455                 460
Tyr Leu Asp Ser Asp Gly Phe Thr Ile Tyr Asn Gln Ile Ser Thr Val
465                 470                 475                 480
Phe Ser Phe Leu Leu Gly Leu Ser Val Ile Pro Phe Ile Trp Asn Val
                485                 490                 495
Phe Lys Ser Trp Arg Tyr Gly Glu Leu Val Thr Val Asp Asp Pro Trp

500                 505                 510
Gly Tyr Gly Asn Ser Leu Glu Trp Ala Thr Ser Cys Pro Pro Pro Arg
        515                 520                 525
His Asn Phe Ala Ser Leu Pro Arg Ile Arg Ser Glu Arg Pro Ala Phe
    530                 535                 540
Glu Leu His Tyr Pro His Met Ile Glu Ser Met Arg Ala Glu Ala His
545                 550                 555                 560
Thr Gly His His Asp Asp Ile Asn Ala Pro Glu Leu Gly Thr Ala Pro
                565                 570                 575
Ala Leu Ala Ser Asp Ser Ser Arg
            580

<210> 3
<211> 4112
<212> DNA
<213> Corynebacterium glutamicum

<220>
<221> CDS
<222> (604)..(1680)

<220>
<221> CDS
<222> (2715)..(3329)

<220>
<221> sig_peptide
<222> (604)..(685)

<220>
<221> mat_peptide
<222> (688)..(1680)

<400> 3
gcatgcatgg caaggcccgc tcgagtgcag gaacgaatgc ttcagcgttc ccatttgcag 60
tcaatatgcc aagaaggccg cacatgattg aaaaatcctc ctgaaataaa aggcgcctta 120
aatcgcaaag aaattttgtt ggaagaaata agacgctgca tttgttaaat ctcgtgtcaa 180
cgatacggcg aagttacatc tgaggtgaaa agggcacgcc aaaattgacg aaagctccct 240
caagcaacgt gcgtcagctg actattgcag cattctcaaa ggttcctgaa aaccagattg 300
atttcctaat acatgcacct tgtaggaacg taggggtaa gggtggggga atttcaaggg 360
caatcaaaag gttgatggtc tgtgacgtgg catacaccaa ttgcctagac tttaggtatt 420

ccacctgagg attcgggcat atcgttgcag ttgaaagaca tttgacgccc ctaaaaacga 480
aacccacgaa gatatttcca ccaaacacaa gatatggaat cggctggcaa ataggctatt 540
ctgcgaagat agaaatgacc gtaaggtctc tggtttttgt gtggacagga aggcagaaca 600
cac gtg gaa cag caa aat aag cgt ggt tta aag cgc aag gcc ctg ctt 648
Val Glu Gln Gln Asn Lys Arg Gly Leu Lys Arg Lys Ala Leu Leu
-25 -20 -15
ggc ggt gtc ttg ggc tta ggt ggc ctc gcc atg gca ggc tgt gaa gtc 696
Gly Gly Val Leu Gly Leu Gly Gly Leu Ala Met Ala Gly Cys Glu Val
-10 -5 -1 1
gcc cct cct ggc ggt gtg ctt gga gat ttc cta cgt atg ggt tgg cct 744
Ala Pro Pro Gly Gly Val Leu Gly Asp Phe Leu Arg Met Gly Trp Pro
                5                   10                  15
gat ggc att acc cct gaa gca gtg gcc atg ggt aac ttc tgg tca tgg 792
Asp Gly Ile Thr Pro Glu Ala Val Ala Met Gly Asn Phe Trp Ser Trp
20 25 30 35
gtc tgg gtt gct gcc tgc atc atc ggc atc atc atg tgg ggt cta ttc 840
Val Trp Val Ala Ala Cys Ile Ile Gly Ile Ile Met Trp Gly Leu Phe
40 45 50
ctc acc gcc atc ttt gcc tgg ggc gca aag agg gct gaa aag cgc ggc 888
Leu Thr Ala Ile Phe Ala Trp Gly Ala Lys Arg Ala Glu Lys Arg Gly
55 60 65
gag ggt gaa ttc cct aag cag ctc cag tac aac gtt cca ctt gag ctc 936
Glu Gly Glu Phe Pro Lys Gln Leu Gln Tyr Asn Val Pro Leu Glu Leu
70 75 80
gtt ctg acg atc gtt ccg atc atc att gtt atg gtg ctg ttc ttc ttc 984
Val Leu Thr Ile Val Pro Ile Ile Ile Val Met Val Leu Phe Phe Phe
                85                  90                  95
acc gtt caa act cag gac aag gtc acc gct ctg gat aag aac cca gag 1032
Thr Val Gln Thr Gln Asp Lys Val Thr Ala Leu Asp Lys Asn Pro Glu
100 105 110 115
gtt acc gtg gac gtc acc gct tac cag tgg aac tgg aag ttc gga tac 1080
Val Thr Val Asp Val Thr Ala Tyr Gln Trp Asn Trp Lys Phe Gly Tyr
120 125 130
tcc gaa att gat ggc tca ctg gca cct ggt gga cag gat tac caa gga 1128
Ser Glu Ile Asp Gly Ser Leu Ala Pro Gly Gly Gln Asp Tyr Gln Gly
135 140 145
agc gac ccg gag cgt cag gca gct gcc gag gct tcc aag aag gat cct 1176
Ser Asp Pro Glu Arg Gln Ala Ala Ala Glu Ala Ser Lys Lys Asp Pro
150 155 160
tct gga gat aac cca att cac ggc aac tca aag tct gac gtt tct tac 1224
Ser Gly Asp Asn Pro Ile His Gly Asn Ser Lys Ser Asp Val Ser Tyr

165                 170                 175
ctt gag ttc aac cga att gaa acc ctc gga acc act gat gaa atc cca   1272
Leu Glu Phe Asn Arg Ile Glu Thr Leu Gly Thr Thr Asp Glu Ile Pro
180                 185                 190                 195
gtg atg gtt ctt cct gtg aac acc cca atc gag ttc aac ctc gca tct   1320
Val Met Val Leu Pro Val Asn Thr Pro Ile Glu Phe Asn Leu Ala Ser
                200                 205                 210
gct gac gtt gca cac tcc ttc tgg gtt cca gag ttc ctc ttc aag cga   1368
Ala Asp Val Ala His Ser Phe Trp Val Pro Glu Phe Leu Phe Lys Arg
            215                 220                 225
gat gct tac gca cac cct gag gca aac aag tcc cag cgt gtc ttc cag   1416
Asp Ala Tyr Ala His Pro Glu Ala Asn Lys Ser Gln Arg Val Phe Gln
        230                 235                 240
att gaa gag atc act gag gaa ggc gca ttc gtt ggt cgc tgt gca gaa   1464
Ile Glu Glu Ile Thr Glu Glu Gly Ala Phe Val Gly Arg Cys Ala Glu
    245                 250                 255
atg tgc ggt act tac cac gca atg atg aac ttc gag ctt cgt gtc gtc   1512
Met Cys Gly Thr Tyr His Ala Met Met Asn Phe Glu Leu Arg Val Val
260                 265                 270                 275
gat cgc gat tcc ttc gct gtg tac atc agc ttc cgt gac tcc aac cca   1560
Asp Arg Asp Ser Phe Ala Val Tyr Ile Ser Phe Arg Asp Ser Asn Pro
                280                 285                 290
gac gca acc aac gct cag gca ctt gag cac att ggt caa gct cct tac   1608
Asp Ala Thr Asn Ala Gln Ala Leu Glu His Ile Gly Gln Ala Pro Tyr
            295                 300                 305
gct act tcc act agc cca ttc gtt tcc gat cgc acc gca acc cgc gac   1656
Ala Thr Ser Thr Ser Pro Phe Val Ser Asp Arg Thr Ala Thr Arg Asp
        310                 315                 320
ggc gaa aac act cag agc aac gct taagaaggag tggcgaaaaa atgaagtctt   1710
Gly Glu Asn Thr Gln Ser Asn Ala
    325                 330
cagcaaaact catgtacggc ccgaccgtat tcatggccgc aatggctgtc atctacatct 1770
tcgcaacaat gcacgttagt gatggcggca gcgttaaagg tgttgagtgg gtcggttctg 1830
tggccctggt cctgtcagca ggtctgacgc ttatgctcgg tgtctacctt cacttcactg 1890
aagtccgcgt agatgttctt ccagaggact gggaagaggc tgaggttgcc gacaaggcag 1950
gaaccctcgg gttcttcagc ccaagctcca tttggccggc agctatgtcc ggtgcggttg 2010
gattccttgc attcggcgtt gtgtacttcc actactggat gatcgcagtt ggtctgatgc 2070
tcctgatctt cacgatcacc aagctcaacc ttcagtacgg cgtgccaaaa gaaaagcact 2130
agtactaaaa ccacatatgc tcaaccctat tggctgtccg atagggttga gcattttgtc 2190
gttttcacag gcttttccaa ctttgatcac cgtgacgaaa ctatcccaac agggcagacc 2250
gaggcgctgt gaaatacgaa aatcggcaaa attgaaaaag ttcccgcaaa atcgagaaaa 2310

gtttctgaaa gcggaaaagt gggggagtgg gcggacatga atcgcattaa gctgcaaaaa 2370
cgccttttat tcgggttcaa ccaaaagttg tatgtggggc tccaatgggg tgaaaaactc 2430
tgtgacgtgt ctctcaggca aaaagctgcg gaaaccactt tgggcacact ctgagagttc 2490
ttcaaaatgg gacgagtgtg atttggggca gattggaact gcctgtgaac cctttaacct 2550
gcaattatgt aactgtcgta aaagggctac gaaaagttcc cggaaggtcg attgaaaagt 2610
ttgcgaattg ggggaaaatt cgcatcaaaa gccgagttca aactttcaat tgaaacgggg 2670
ggcttgaagt gactttggcc aaccaaacag ccatactaga tagc gtg acg agc gca 2726
Val Thr Ser Ala
1
gtt gga aat aca ggt atg gca gca cca caa cgt gtt gcg gca ctg aac 2774
Val Gly Asn Thr Gly Met Ala Ala Pro Gln Arg Val Ala Ala Leu Asn
5 10 15 20
cgt ccg aat atg gtc agt gtc ggc acc att gtg ttc ctg tct cag gaa 2822
Arg Pro Asn Met Val Ser Val Gly Thr Ile Val Phe Leu Ser Gln Glu
25 30 35
tta atg ttc ttc gcc ggg cta ttc gcg atg tac ttc gtg tcc cgt gcg 2870
Leu Met Phe Phe Ala Gly Leu Phe Ala Met Tyr Phe Val Ser Arg Ala
40 45 50
aac gga ctg gca aat gga tca tgg gga gag cag aca gat cac ctc aac 2918
Asn Gly Leu Ala Asn Gly Ser Trp Gly Glu Gln Thr Asp His Leu Asn
55 60 65
gtg ccc tac gca ctg ttg att acg gtc att ctg gtg tct tcc tca gtg 2966
Val Pro Tyr Ala Leu Leu Ile Thr Val Ile Leu Val Ser Ser Ser Val
70 75 80
act tgc cag ttc gga gtt ttt gcg gct gaa agg ggt gac gtt tac ggc 3014
Thr Cys Gln Phe Gly Val Phe Ala Ala Glu Arg Gly Asp Val Tyr Gly
85 90 95 100
ctc cgc aag tgg ttc ttg gtc acg att atc ctc gga tca atc ttc gtg 3062
Leu Arg Lys Trp Phe Leu Val Thr Ile Ile Leu Gly Ser Ile Phe Val
105 110 115
atc gga cag ggc tac gag tac atc act ctc gta ggt cac gga ctt aca 3110
Ile Gly Gln Gly Tyr Glu Tyr Ile Thr Leu Val Gly His Gly Leu Thr
120 125 130
atc cag agc agt gtc tac gga tcg gca ttc ttt att aca acc ggt ttc 3158
Ile Gln Ser Ser Val Tyr Gly Ser Ala Phe Phe Ile Thr Thr Gly Phe
135 140 145
cac gca ctg cac gtg atc gcg ggt gtt atg gcc ttc gtt gtg gtt ctt 3206
His Ala Leu His Val Ile Ala Gly Val Met Ala Phe Val Val Val Leu
150 155 160
atg aga atc cat aag tcg aag ttc act ccg gca cag gca acc gca gca 3254
Met Arg Ile His Lys Ser Lys Phe Thr Pro Ala Gln Ala Thr Ala Ala

165                    170                    175                    180
atg gtt gtg tct tat tac tgg cac ttc gtt gac gtg gtc tgg atc ggc    3302
Met Val Val Ser Tyr Tyr Trp His Phe Val Asp Val Val Trp Ile Gly
                185                    190                    195
ctc ttc atc act att tac ttc att cag taggcagtaa ggaatcctca          3349
Leu Phe Ile Thr Ile Tyr Phe Ile Gln
            200                    205
acgttgttga ggttccctat gcccttcact tccacagtcg agattcaaag ggaaatgatg 3409
gaaaccaacc cgcagacccc agagggaaat agcatggcta aaccctctgc taagaaggtc 3469
aagaatcgcc gcaaggtccg gcgcaccgtc gcaggtgcat tggctctgac cattggactg 3529
agcggagcag gaatcctcgc aaccgcgatc actccagatg ctcaagttgc taccgctcag 3589
cgtgacgatc aggcacttat ctccgagggt aaagacctct acgatgtcgc ctgcatcacc 3649
tgccacggcg taaacctcca aggtgttgag gaccgcggtc cttccctcgt aggtgttggc 3709
gaaggcgcag tgtacttcca agttcactcc ggccgtatgc caatactgcg taacgaggct 3769
caggctgagc gcaaggctcc tcgttacacc gaggcacaga cccttgcgat cgctgcatat 3829
gttgcagcta atggcggtgg cccaggactc gtttacaacg aggacggcac cctcgccatg 3889
gaggagctcc gtggcgaaaa ctacgacgga cagattacct ccgccgacgt cgctcgcggc 3949
ggagatctgt tccgcctgaa ctgtgcatcc tgccacaact tcactggtcg tggtggcgca 4009
ctgtcctctg gtaagtacgc accaaacctg gatgctgcaa acgagcagga aatctaccag 4069
gctatgctta ccggtcctca gaacatgcct aagttctccg atc                   4112

<210> 4
<211> 359
<212> PRT
<213> Corynebacterium glutamicum

<400> 4
Val Glu Gln Gln Asn Lys Arg Gly Leu Lys Arg Lys Ala Leu Leu Gly
            -25                    -20                    -15
Gly Val Leu Gly Leu Gly Gly Leu Ala Met Ala Gly Cys Glu Val Ala
        -10                    -5                    -1   1
Pro Pro Gly Gly Val Leu Gly Asp Phe Leu Arg Met Gly Trp Pro Asp
  5                    10                    15                    20
Gly Ile Thr Pro Glu Ala Val Ala Met Gly Asn Phe Trp Ser Trp Val
                25                    30                    35
Trp Val Ala Ala Cys Ile Ile Gly Ile Ile Met Trp Gly Leu Phe Leu
            40                    45                    50
Thr Ala Ile Phe Ala Trp Gly Ala Lys Arg Ala Glu Lys Arg Gly Glu
        55                    60                    65
Gly Glu Phe Pro Lys Gln Leu Gln Tyr Asn Val Pro Leu Glu Leu Val
    70                    75                    80

Leu Thr Ile Val Pro Ile Ile Ile Val Met Val Leu Phe Phe Phe Thr
85                  90                  95                  100
Val Gln Thr Gln Asp Lys Val Thr Ala Leu Asp Lys Asn Pro Glu Val
                105                 110                 115
Thr Val Asp Val Thr Ala Tyr Gln Trp Asn Trp Lys Phe Gly Tyr Ser
            120                 125                 130
Glu Ile Asp Gly Ser Leu Ala Pro Gly Gly Gln Asp Tyr Gln Gly Ser
        135                 140                 145
Asp Pro Glu Arg Gln Ala Ala Ala Glu Ala Ser Lys Lys Asp Pro Ser
    150                 155                 160
Gly Asp Asn Pro Ile His Gly Asn Ser Lys Ser Asp Val Ser Tyr Leu
165                 170                 175                 180
Glu Phe Asn Arg Ile Glu Thr Leu Gly Thr Thr Asp Glu Ile Pro Val
                185                 190                 195
Met Val Leu Pro Val Asn Thr Pro Ile Glu Phe Asn Leu Ala Ser Ala
            200                 205                 210
Asp Val Ala His Ser Phe Trp Val Pro Glu Phe Leu Phe Lys Arg Asp
        215                 220                 225
Ala Tyr Ala His Pro Glu Ala Asn Lys Ser Gln Arg Val Phe Gln Ile
    230                 235                 240
Glu Glu Ile Thr Glu Glu Gly Ala Phe Val Gly Arg Cys Ala Glu Met
245                 250                 255                 260
Cys Gly Thr Tyr His Ala Met Met Asn Phe Glu Leu Arg Val Val Asp
                265                 270                 275
Arg Asp Ser Phe Ala Val Tyr Ile Ser Phe Arg Asp Ser Asn Pro Asp
            280                 285                 290
Ala Thr Asn Ala Gln Ala Leu Glu His Ile Gly Gln Ala Pro Tyr Ala
        295                 300                 305
Thr Ser Thr Ser Pro Phe Val Ser Asp Arg Thr Ala Thr Arg Asp Gly
    310                 315                 320
Glu Asn Thr Gln Ser Asn Ala
325                 330

<210> 5
<211> 205
<212> PRT
<213> Corynebacterium glutamicum

<400> 5
Val Thr Ser Ala Val Gly Asn Thr Gly Met Ala Ala Pro Gln Arg Val
1               5                   10                  15

Ala Ala Leu Asn Arg Pro Asn Met Val Ser Val Gly Thr Ile Val Phe
            20                  25                  30
Leu Ser Gln Glu Leu Met Phe Phe Ala Gly Leu Phe Ala Met Tyr Phe
        35                  40                  45
Val Ser Arg Ala Asn Gly Leu Ala Asn Gly Ser Trp Gly Glu Gln Thr
    50                  55                  60
Asp His Leu Asn Val Pro Tyr Ala Leu Leu Ile Thr Val Ile Leu Val
65                  70                  75                  80
Ser Ser Ser Val Thr Cys Gln Phe Gly Val Phe Ala Ala Glu Arg Gly
                85                  90                  95
Asp Val Tyr Gly Leu Arg Lys Trp Phe Leu Val Thr Ile Ile Leu Gly
            100                 105                 110
Ser Ile Phe Val Ile Gly Gln Gly Tyr Glu Tyr Ile Thr Leu Val Gly
        115                 120                 125
His Gly Leu Thr Ile Gln Ser Ser Val Tyr Gly Ser Ala Phe Phe Ile
    130                 135                 140
Thr Thr Gly Phe His Ala Leu His Val Ile Ala Gly Val Met Ala Phe
145                 150                 155                 160
Val Val Val Leu Met Arg Ile His Lys Ser Lys Phe Thr Pro Ala Gln
                165                 170                 175
Ala Thr Ala Ala Met Val Val Ser Tyr Tyr Trp His Phe Val Asp Val
            180                 185                 190
Val Trp Ile Gly Leu Phe Ile Thr Ile Tyr Phe Ile Gln
        195                 200                 205

<210> 6
<211> 4683
<212> DNA
<213> Corynebacterium glutamicum

<220>
<221> CDS
<222> (276)..(1124)

<220>
<221> CDS
<222> (1172)..(2347)

<400> 6
cacgtgatcg cgggtgttat ggccttcgtt gtggttctta tgagaatcca taagtcgaag 60
ttcactccgg cacaggcaac cgcagcaatg gttgtgtctt attactggca cttcgttgac 120

gtggtctgga tcggcctctt catcactatt tacttcattc agtaggcagt aaggaatcct 180
caacgttgtt gaggttccct atgcccttca cttccacagt cgagattcaa agggaaatga 240
tggaaaccaa cccgcagacc ccagaggaaa atagc atg gct aaa ccc tct gct 293
Met Ala Lys Pro Ser Ala
1               5
aag aag gtc aag aat cgc cgc aag gtc cgg cgc acc gtc gca ggt gca 341
Lys Lys Val Lys Asn Arg Arg Lys Val Arg Arg Thr Val Ala Gly Ala
            10                  15                  20
ttg gct ctg acc att gga ctg agc gga gca gga atc ctc gca acc gcg 389
Leu Ala Leu Thr Ile Gly Leu Ser Gly Ala Gly Ile Leu Ala Thr Ala
        25                  30                  35
atc act cca gat gct caa gtt gct acc gct cag cgt gac gat cag gca 437
Ile Thr Pro Asp Ala Gln Val Ala Thr Ala Gln Arg Asp Asp Gln Ala
    40                  45                  50
ctt atc tcc gag ggt aaa gac ctc tac gct gtc gcc tgc atc acc tgt 485
Leu Ile Ser Glu Gly Lys Asp Leu Tyr Ala Val Ala Cys Ile Thr Cys
55                  60                  65                  70
cac ggc gta aac ctc caa ggt gtt gag gac cgc ggt cct tcc ctc gta 533
His Gly Val Asn Leu Gln Gly Val Glu Asp Arg Gly Pro Ser Leu Val
                75                  80                  85
ggt gtt ggc gaa ggc gca gtg tac ttc caa gtt cac tcc ggc cgt atg 581
Gly Val Gly Glu Gly Ala Val Tyr Phe Gln Val His Ser Gly Arg Met
            90                  95                  100
cca atg ctg cgt aac gag gct cag gct gag cgc aag gct cct cgt tac 629
Pro Met Leu Arg Asn Glu Ala Gln Ala Glu Arg Lys Ala Pro Arg Tyr
        105                 110                 115
acc gag gca cag acc ctt gcg atc gct gca tat gtt gca gct aat ggc 677
Thr Glu Ala Gln Thr Leu Ala Ile Ala Ala Tyr Val Ala Ala Asn Gly
    120                 125                 130
ggt ggc cca gga ctc gtt tac aac gag gac ggc acc ctg gcc atg gag 725
Gly Gly Pro Gly Leu Val Tyr Asn Glu Asp Gly Thr Leu Ala Met Glu
135                 140                 145                 150
gag ctc cgt ggc gaa aac tac gac gga cag att acc tcc gcc gac gtc 773
Glu Leu Arg Gly Glu Asn Tyr Asp Gly Gln Ile Thr Ser Ala Asp Val
                155                 160                 165
gct cgc ggc gga gat ctg ttc cgc ctg aac tgt gca tcc tgc cac aac 821
Ala Arg Gly Gly Asp Leu Phe Arg Leu Asn Cys Ala Ser Cys His Asn
            170                 175                 180
ttc act ggt cgt ggt ggc gca ctg tcc tct ggt aag tac gca cca aac 869
Phe Thr Gly Arg Gly Gly Ala Leu Ser Ser Gly Lys Tyr Ala Pro Asn
        185                 190                 195

ctg gat gct gca aac gag cag gaa atc tac cag gct atg ctt acc ggt 917
Leu Asp Ala Ala Asn Glu Gln Glu Ile Tyr Gln Ala Met Leu Thr Gly
    200                 205                 210
cct cag aac atg cct aag ttc tcc gat cgt cag ctc tcc gca gat gag 965
Pro Gln Asn Met Pro Lys Phe Ser Asp Arg Gln Leu Ser Ala Asp Glu
215                 220                 225                 230
aag aag gac atc atc gcc ttc atc aag tcc acc aag gag act cca tca 1013
Lys Lys Asp Ile Ile Ala Phe Ile Lys Ser Thr Lys Glu Thr Pro Ser
                235                 240                 245
cct ggt ggt tac tca ctc ggt agc ttg ggc cca gtg gct gag ggt ctg 1061
Pro Gly Gly Tyr Ser Leu Gly Ser Leu Gly Pro Val Ala Glu Gly Leu
            250                 255                 260
ttc atg tgg gta ttc ggc atc ttg gtc ctc gtg gcc gcc gct atg tgg 1109
Phe Met Trp Val Phe Gly Ile Leu Val Leu Val Ala Ala Ala Met Trp
        265                 270                 275
att gga tca cgt tca tgagtaacaa caacgacaag cagtacacaa cccaagaact 1164
Ile Gly Ser Arg Ser
    280
caacgcg atg agc aat gag gat ctt gca cga ctt ggt aca gag ctg gac 1213
Met Ser Asn Glu Asp Leu Ala Arg Leu Gly Thr Glu Leu Asp
1 5 10
gac gtt acc att gca tac cgc aag gaa cgt ttc cca atc cct aat gac 1261
Asp Val Thr Ile Ala Tyr Arg Lys Glu Arg Phe Pro Ile Pro Asn Asp
15 20 25 30
cca gct gag aag cgc gct gca cgt tca gtt act ttc tgg cta gtc ctc 1309
Pro Ala Glu Lys Arg Ala Ala Arg Ser Val Thr Phe Trp Leu Val Leu
35 40 45
ggc atc att ggt gga ctt gga ttc ctg gct acc tac att ttc tgg cct 1357
Gly Ile Ile Gly Gly Leu Gly Phe Leu Ala Thr Tyr Ile Phe Trp Pro
50 55 60
tgg gag tac aag gca cac gga gat gaa ggt ctc ctg gcg tac acc ttg 1405
Trp Glu Tyr Lys Ala His Gly Asp Glu Gly Leu Leu Ala Tyr Thr Leu
65 70 75
tac acc cca atg ctg ggt att act tcc ggt ctt tgc atc ctg tcc ctg 1453
Tyr Thr Pro Met Leu Gly Ile Thr Ser Gly Leu Cys Ile Leu Ser Leu
80 85 90
gga ttt gca gtt gtc ctt tat gtc aag aag ttc att cca gag gaa atc 1501
Gly Phe Ala Val Val Leu Tyr Val Lys Lys Phe Ile Pro Glu Glu Ile
95 100 105 110
gca gta cag cgt cgc cac gac ggt cct tct gaa gaa gtt gac cgc cgc 1549
Ala Val Gln Arg Arg His Asp Gly Pro Ser Glu Glu Val Asp Arg Arg

115 120 125
acc atc gtt gca ctt ctc aat gac tct tgg cag acc tct act ctt ggt 1597
Thr Ile Val Ala Leu Leu Asn Asp Ser Trp Gln Thr Ser Thr Leu Gly
130 135 140
cgt cgc aag ctg atc atg gga ctt gca ggt ggc gga gca gta ctg gcc 1645
Arg Arg Lys Leu Ile Met Gly Leu Ala Gly Gly Gly Ala Val Leu Ala
145 150 155
ggc ctg acc atc atc gct cca atg ggc ggt atg atc aag aat cct tgg 1693
Gly Leu Thr Ile Ile Ala Pro Met Gly Gly Met Ile Lys Asn Pro Trp
160 165 170
aag cct aag gaa ggc cca atg gac gtt cag ggt gac ggc acc cta tgg 1741
Lys Pro Lys Glu Gly Pro Met Asp Val Gln Gly Asp Gly Thr Leu Trp
175 180 185 190
act tcc ggt tgg aca ctc att gag aac gac gtc aag gtt tac ctc ggc 1789
Thr Ser Gly Trp Thr Leu Ile Glu Asn Asp Val Lys Val Tyr Leu Gly
195 200 205
cgt gac act gca gca att gcg gag tca cac acc gat gca acc ggt gag 1837
Arg Asp Thr Ala Ala Ile Ala Glu Ser His Thr Asp Ala Thr Gly Glu
210 215 220
cac tgg tca acc act ggt gtt tcc cgc ctg gtt cgt atg cgc cca gaa 1885
His Trp Ser Thr Thr Gly Val Ser Arg Leu Val Arg Met Arg Pro Glu
225 230 235
gat ctg gca gca gca tcc atg gaa act gtc ttc cca ctt cca gct gaa 1933
Asp Leu Ala Ala Ala Ser Met Glu Thr Val Phe Pro Leu Pro Ala Glu
140 245 250
atg gtg aac gac ggt gct gaa tac gat cct gcg aag gac gtc tac gag 1981
Met Val Asn Asp Gly Ala Glu Tyr Asp Pro Ala Lys Asp Val Tyr Glu
255 260 265 270
cac caa atg cac tcg gtg cac ggc cca cgt aac gca gtt atg ttg atc 2029
His Gln Met His Ser Val His Gly Pro Arg Asn Ala Val Met Leu Ile
275 280 285
cgt ctc cgt acc gct gac gct gaa aag gtt atc gaa cgc gaa ggc cag 2077
Arg Leu Arg Thr Ala Asp Ala Glu Lys Val Ile Glu Arg Glu Gly Gln
290 295 300
gag tcc ttc cac tac ggt gac tac tac gct tac tcc aag att tgt aca 2125
Glu Ser Phe His Tyr Gly Asp Tyr Tyr Ala Tyr Ser Lys Ile Cys Thr
305 310 315
cac att ggt tgt cca acc tca ctg tac gag gct cag acc aac cgt att 2173
His Ile Gly Cys Pro Thr Ser Leu Tyr Glu Ala Gln Thr Asn Arg Ile
320 325 330
ctg tgc cca tgt cac cag tcg cag ttt gac gca ttg cac tac gga aag 2221

Leu Cys Pro Cys His Gln Ser Gln Phe Asp Ala Leu His Tyr Gly Lys
335                 340                 345                 350
cca gtc ttc gga cct gct gcc cgt gcg ctg cca cag ctg cca att acc    2269
Pro Val Phe Gly Pro Ala Ala Arg Ala Leu Pro Gln Leu Pro Ile Thr
                355                 360                 365
gtt gat gaa gag ggc tac ctc atc gcc gct ggt aac ttc att gag cca    2317
Val Asp Glu Glu Gly Tyr Leu Ile Ala Ala Gly Asn Phe Ile Glu Pro
            370                 375                 380
ctc ggc cct gca ttc tgg gag cgt aag tca tgagtctagc taccgtggga    2367
Leu Gly Pro Ala Phe Trp Glu Arg Lys Ser
        385                 390
aacaatcttg attcccgtta caccatggcg tcgggtatcc gtcgccagat caacaaggtc 2427
ttcccaactc actggtcctt catgctcggc gagattgcgc tttacagctt catcgtcttg 2487
ctgctgactg gtgtctacct gaccctgttc ttcgacccat caatcaccaa ggtcatttat 2547
gacggcggct acctcccact gaacggtgtg gagatgtccc gtgcatacgc aactgcgttg 2607
gatatttcct tcgaagttcg cggtggtctg ttcatccgcc agatgcacca ctgggcagcc 2667
ctgctgttcg ttgtatccat gctggttcac atgctccgta ttttcttcac cggtgcgttc 2727
cgtcgcccac gtgaagcaaa ctggatcatc ggtgttgttc tgatcatcct gggtatggct 2787
gaaggcttca tgggttactc cctgcctgat gacctgctct ctggtgttgg tcttcgaatc 2847
atgtccgcca tcatcgttgg tcttccgatc atcggtacct ggatgcactg gctgatcttc 2907
ggtggagact tcccatccga tctgatgctg gaccgcttct acatcgcaca cgttctaatc 2967
atcccagcta tcctgcttgg cttgatcgca gctcacctgg cacttgtttg gtaccagaag 3027
cacacccagt tcccaggcgc tggccgcact gagaacaacg tggtcggtat ccgaatcatg 3087
cctctgttcg cagttaaggc tgttgctttc ggcctcatcg tcttcggctt cctcgcactg 3147
cttgctggtg tcaccaccat taacgcaatt tggaatcttg gaccgtacaa cccttcacag 3207
gtgtctgctg gttcccagcc tgacgtttac atgctgtgga cagatggtgc tgctcgtgtc 3267
atgccggcat gggagctcta cctcggtaac tacaccatcc cagcagtctt ctgggttgct 3327
gtgatgctgg gtatcctcgt ggttctgctt gtgacttacc cattcattga gcgtaagttc 3387
actggcgacg atgcacacca caacttgctg cagcgtcctc gcgatgttcc agtccgcacc 3447
tcactcggtg tcatggcgct tgtcttctac atcctgctta ccgtttctgg tggtaacgat 3507
gtttacgcaa tgcagttcca tgtttcactg aacgcgatga cctggatcgg tcgtatcggc 3567
ctcatcgttg gaccagctat tgcatacttc atcacttacc gactgtgcat cggcttgcag 3627
cgctctgacc gcgaggtcct ggagcacggc atcgagaccg gtatcatcaa gcagatgcca 3687
aatggtgcct tcattgaagt tcaccagcca cttggcccag ttgatgacca tggtcaccca 3747
atcccactgc catacgctgg cgctgcggtt ccaaagcaga tgaaccagct tggttacgct 3807
gaggttgaaa cccgcggtgg attcttcgga cctgatccag aagacatccg tgcgaaggct 3867
aaggaaattg agcacgcaaa ccacattgag gaagcgaaca ctcttcgtgc actcaacgag 3927
gcaaacattg agcgtgacaa gaatgagggc aagaactagt ttctaggact tcatctctga 3987
aactccccgc tgtagggacc tgaatcgaaa ggtctccgca tcggggagtt tttctctatt 4047
cagacgaggc taaagataag ggagagggct ctttaacaca cgaggagtgg cgtagagcct 4107
gtagttgcct tatatgtagc ttgtggcggc gtgaagcaac gtgcaggcgc gtggaaagcg 4167

tagagttttc ttctccttat atataaggag tgttcttcgt cgtgagcatt acgccctagg 4227
tcgcctgggg tcggtgtcca acttcgctca aattccgctc aaaatccagc tcggtgtggc 4287
ttaagaattg ttgtcataac tccaaatctc aaagcataag ccgtcaatgg tgattaatgt 4347
cacatggtga gatcattgct gaaactggtg ccgatttttc ggctctgtga aaacgatttg 4407
actactggaa gtttcctgaa attgcaggtc atctagcttt ctcagggttc taggggagaa 4467
cccttagtgg ttggggtctg agtggaggac ttgcgtctcg gtcaaattaa tccgcgataa 4527
cggttcgata acgaccaatt ttttcgcttg ggctagacaa gtgttgttgc ggtttcgtaa 4587
ccttattgag acattgcggg acggacaccg aatttccgcc agcattacag aaacaaatag 4647
acgcttaatc gcaagcatag tttagagaaa ttcttt 4683

<210> 7
<211> 283
<212> PRT
<213> Corynebacterium glutamicum

<400> 7
Met Ala Lys Pro Ser Ala Lys Lys Val Lys Asn Arg Arg Lys Val Arg
1               5                   10                  15
Arg Thr Val Ala Gly Ala Leu Ala Leu Thr Ile Gly Leu Ser Gly Ala
            20                  25                  30
Gly Ile Leu Ala Thr Ala Ile Thr Pro Asp Ala Gln Val Ala Thr Ala
        35                  40                  45
Gln Arg Asp Asp Gln Ala Leu Ile Ser Glu Gly Lys Asp Leu Tyr Ala
    50                  55                  60
Val Ala Cys Ile Thr Cys His Gly Val Asn Leu Gln Gly Val Glu Asp
65                  70                  75                  80
Arg Gly Pro Ser Leu Val Gly Val Gly Glu Gly Ala Val Tyr Phe Gln
                85                  90                  95
Val His Ser Gly Arg Met Pro Met Leu Arg Asn Glu Ala Gln Ala Glu
            100                 105                 110
Arg Lys Ala Pro Arg Tyr Thr Glu Ala Gln Thr Leu Ala Ile Ala Ala
        115                 120                 125
Tyr Val Ala Ala Asn Gly Gly Gly Pro Gly Leu Val Tyr Asn Glu Asp
    130                 135                 140
Gly Thr Leu Ala Met Glu Glu Leu Arg Gly Glu Asn Tyr Asp Gly Gln
145                 150                 155                 160
Ile Thr Ser Ala Asp Val Ala Arg Gly Gly Asp Leu Phe Arg Leu Asn
                165                 170                 175
Cys Ala Ser Cys His Asn Phe Thr Gly Arg Gly Gly Ala Leu Ser Ser
            180                 185                 190
Gly Lys Tyr Ala Pro Asn Leu Asp Ala Ala Asn Glu Gln Glu Ile Tyr

195                 200                 205
Gln Ala Met Leu Thr Gly Pro Gln Asn Met Pro Lys Phe Ser Asp Arg
    210                 215                 220
Gln Leu Ser Ala Asp Glu Lys Lys Asp Ile Ile Ala Phe Ile Lys Ser
225                 230                 235                 240
Thr Lys Glu Thr Pro Ser Pro Gly Gly Tyr Ser Leu Gly Ser Leu Gly
                245                 250                 255
Pro Val Ala Glu Gly Leu Phe Met Trp Val Phe Gly Ile Leu Val Leu
            260                 265                 270
Val Ala Ala Ala Met Trp Ile Gly Ser Arg Ser
        275                 280

<210> 8
<211> 392
<212> PRT
<213> Corynebacterium glutamicum

<400> 8
Met Ser Asn Glu Asp Leu Ala Arg Leu Gly Thr Glu Leu Asp Asp Val
  1               5                  10                  15
Thr Ile Ala Tyr Arg Lys Glu Arg Phe Pro Ile Pro Asn Asp Pro Ala
             20                  25                  30
Glu Lys Arg Ala Ala Arg Ser Val Thr Phe Trp Leu Val Leu Gly Ile
         35                  40                  45
Ile Gly Gly Leu Gly Phe Leu Ala Thr Tyr Ile Phe Trp Pro Trp Glu
     50                  55                  60
Tyr Lys Ala His Gly Asp Glu Gly Leu Leu Ala Tyr Thr Leu Tyr Thr
 65                  70                  75                  80
Pro Met Leu Gly Ile Thr Ser Gly Leu Cys Ile Leu Ser Leu Gly Phe
                 85                  90                  95
Ala Val Val Leu Tyr Val Lys Lys Phe Ile Pro Glu Glu Ile Ala Val
            100                 105                 110
Gln Arg Arg His Asp Gly Pro Ser Glu Glu Val Asp Arg Arg Thr Ile
        115                 120                 125
Val Ala Leu Leu Asn Asp Ser Trp Gln Thr Ser Thr Leu Gly Arg Arg
    130                 135                 140
Lys Leu Ile Met Gly Leu Ala Gly Gly Gly Ala Val Leu Ala Gly Leu
145                 150                 155                 160
Thr Ile Ile Ala Pro Met Gly Gly Met Ile Lys Asn Pro Trp Lys Pro
                165                 170                 175
Lys Glu Gly Pro Met Asp Val Gln Gly Asp Gly Thr Leu Trp Thr Ser

180                 185                 190
Gly Trp Thr Leu Ile Glu Asn Asp Val Lys Val Tyr Leu Gly Arg Asp
        195                 200                 205
Thr Ala Ala Ile Ala Glu Ser His Thr Asp Ala Thr Gly Glu His Trp
    210                 215                 220
Ser Thr Thr Gly Val Ser Arg Leu Val Arg Met Arg Pro Glu Asp Leu
225                 230                 235                 240
Ala Ala Ala Ser Met Glu Thr Val Phe Pro Leu Pro Ala Glu Met Val
                245                 250                 255
Asn Asp Gly Ala Glu Tyr Asp Pro Ala Lys Asp Val Tyr Glu His Gln
            260                 265                 270
Met His Ser Val His Gly Pro Arg Asn Ala Val Met Leu Ile Arg Leu
        275                 280                 285
Arg Thr Ala Asp Ala Glu Lys Val Ile Glu Arg Glu Gly Gln Glu Ser
    290                 295                 300
Phe His Tyr Gly Asp Tyr Tyr Ala Tyr Ser Lys Ile Cys Thr His Ile
305                 310                 315                 320
Gly Cys Pro Thr Ser Leu Tyr Glu Ala Gln Thr Asn Arg Ile Leu Cys
                325                 330                 335
Pro Cys His Gln Ser Gln Phe Asp Ala Leu His Tyr Gly Lys Pro Val
            340                 345                 350
Phe Gly Pro Ala Ala Arg Ala Leu Pro Gln Leu Pro Ile Thr Val Asp
        355                 360                 365
Glu Glu Gly Tyr Leu Ile Ala Ala Gly Asn Phe Ile Glu Pro Leu Gly
    370                 375                 380
Pro Ala Phe Trp Glu Arg Lys Ser
385                 390

<210> 9
<211> 4683
<212> DNA
<213> Corynebacterium glutamicum

<220>
<221> CDS
<222> (2347)..(3963)

<400> 9
cacgtgatcg cgggtgttat ggccttcgtt gtggttctta tgagaatcca taagtcgaag 60
ttcactccgg cacaggcaac cgcagcaatg gttgtgtctt attactggca cttcgttgac 120
gtggtctgga tcggcctctt catcactatt tacttcattc agtaggcagt aaggaatcct 180

caacgttgtt gaggttccct atgcccttca cttccacagt cgagattcaa agggaaatga 240
tggaaaccaa cccgcagacc ccagaggaaa atagcatggc taaaccctct gctaagaagg 300
tcaagaatcg ccgcaaggtc cggcgcaccg tcgcaggtgc attggctctg accattggac 360
tgagcggagc aggaatcctc gcaaccgcga tcactccaga tgctcaagtt gctaccgctc 420
agcgtgacga tcaggcactt atctccgagg gtaaagacct ctacgctgtc gcctgcatca 480
cctgtcacgg cgtaaacctc caaggtgttg aggaccgcgg tccttccctc gtaggtgttg 540
gcgaaggcgc agtgtacttc caagttcact ccggccgtat gccaatgctg cgtaacgagg 600
ctcaggctga gcgcaaggct cctcgttaca ccgaggcaca gacccttgcg atcgctgcat 660
atgttgcagc taatggcggt ggcccaggac tcgtttacaa cgaggacggc accctggcca 720
tggaggagct ccgtggcgaa aactacgacg gacagattac ctccgccgac gtcgctcgcg 780
gcggagatct gttccgcctg aactgtgcat cctgccacaa cttcactggt cgtggtggcg 840
cactgtcctc tggtaagtac gcaccaaacc tggatgctgc aaacgagcag gaaatctacc 900
aggctatgct taccggtcct cagaacatgc ctaagttctc cgatcgtcag ctctccgcag 960
atgagaagaa ggacatcatc gccttcatca agtccaccaa ggagactcca tcacctggtg 1020
gttactcact cggtagcttg ggcccagtgg ctgagggtct gttcatgtgg gtattcggca 1080
tcttggtcct cgtggccgcc gctatgtgga ttggatcacg ttcatgagta acaacaacga 1140
caagcagtac acaacccaag aactcaacgc gatgagcaat gaggatcttg cacgacttgg 1200
tacagagctg gacgacgtta ccattgcata ccgcaaggaa cgtttcccaa tccctaatga 1260
cccagctgag aagcgcgctg cacgttcagt tactttctgg ctagtcctcg gcatcattgg 1320
tggacttgga ttcctggcta cctacatttt ctggccttgg gagtacaagg cacacggaga 1380
tgaaggtctc ctggcgtaca ccttgtacac cccaatgctg ggtattactt ccggtctttg 1440
catcctgtcc ctgggatttg cagttgtcct ttatgtcaag aagttcattc cagaggaaat 1500
cgcagtacag cgtcgccacg acggtccttc tgaagaagtt gaccgccgca ccatcgttgc 1560
acttctcaat gactcttggc agacctctac tcttggtcgt cgcaagctga tcatgggact 1620
tgcaggtggc ggagcagtac tggccggcct gaccatcatc gctccaatgg gcggtatgat 1680
caagaatcct tggaagccta aggaaggccc aatggacgtt cagggtgacg gcaccctatg 1740
gacttccggt tggacactca ttgagaacga cgtcaaggtt tacctcggcc gtgacactgc 1800
agcaattgcg gagtcacaca ccgatgcaac cggtgagcac tggtcaacca ctggtgtttc 1860
ccgcctggtt cgtatgcgcc cagaagatct ggcagcagca tccatggaaa ctgtcttccc 1920
acttccagct gaaatggtga acgacggtgc tgaatacgat cctgcgaagg acgtctacga 1980
gcaccaaatg cactcggtgc acggcccacg taacgcagtt atgttgatcc gtctccgtac 2040
cgctgacgct gaaaaggtta tcgaacgcga aggccaggag tccttccact acggtgacta 2100
ctacgcttac tccaagattt gtacacacat tggttgtcca acctcactgt acgaggctca 2160
gaccaaccgt attctgtgcc catgtcacca gtcgcagttt gacgcattgc actacggaaa 2220
gccagtcttc ggacctgctg cccgtgcgct gccacagctg ccaattaccg ttgatgaaga 2280
gggctacctc atcgccgctg gtaacttcat tgagccactc ggccctgcat tctgggagcg 2340
taagtc atg agt cta gct acc gtg gga aac aat ctt gat tcc cgt tac 2388
Met Ser Leu Ala Thr Val Gly Asn Asn Leu Asp Ser Arg Tyr
1               5                   10
acc atg gcg tcg ggt atc cgt cgc cag atc aac aag gtc ttc cca act 2436
Thr Met Ala Ser Gly Ile Arg Arg Gln Ile Asn Lys Val Phe Pro Thr

15                  20                  25                  30
cac tgg tcc ttc atg ctc ggc gag att gcg ctt tac agc ttc atc gtc 2484
His Trp Ser Phe Met Leu Gly Glu Ile Ala Leu Tyr Ser Phe Ile Val
                35                  40                  45
ttg ctg ctg act ggt gtc tac ctg acc ctg ttc ttc gac cca tca atc 2532
Leu Leu Leu Thr Gly Val Tyr Leu Thr Leu Phe Phe Asp Pro Ser Ile
            50                  55                  60
acc aag gtc att tat gac ggc ggc tac ctc cca ctg aac ggt gtg gag 2580
Thr Lys Val Ile Tyr Asp Gly Gly Tyr Leu Pro Leu Asn Gly Val Glu
        65                  70                  75
atg tcc cgt gca tac gca act gcg ttg gat att tcc ttc gaa gtt cgc 2628
Met Ser Arg Ala Tyr Ala Thr Ala Leu Asp Ile Ser Phe Glu Val Arg
    80                  85                  90
ggt ggt ctg ttc atc cgc cag atg cac cac tgg gca gcc ctg ctg ttc 2676
Gly Gly Leu Phe Ile Arg Gln Met His His Trp Ala Ala Leu Leu Phe
95                  100                 105                 110
gtt gta tcc atg ctg gtt cac atg ctc cgt att ttc ttc acc ggt gcg 2724
Val Val Ser Met Leu Val His Met Leu Arg Ile Phe Phe Thr Gly Ala
                115                 120                 125
ttc cgt cgc cca cgt gaa gca aac tgg atc atc ggt gtt gtt ctg atc 2772
Phe Arg Arg Pro Arg Glu Ala Asn Trp Ile Ile Gly Val Val Leu Ile
            130                 135                 140
atc ctg ggt atg gct gaa ggc ttc atg ggt tac tcc ctg cct gat gac 2820
Ile Leu Gly Met Ala Glu Gly Phe Met Gly Tyr Ser Leu Pro Asp Asp
        145                 150                 155
ctg ctc tct ggt gtt ggt ctt cga atc atg tcc gcc atc atc gtt ggt 2868
Leu Leu Ser Gly Val Gly Leu Arg Ile Met Ser Ala Ile Ile Val Gly
    160                 165                 170
ctt ccg atc atc ggt acc tgg atg cac tgg ctg atc ttc ggt gga gac 2916
Leu Pro Ile Ile Gly Thr Trp Met His Trp Leu Ile Phe Gly Gly Asp
175                 180                 185                 190
ttc cca tcc gat ctg atg ctg gac cgc ttc tac atc gca cac gtt cta 2964
Phe Pro Ser Asp Leu Met Leu Asp Arg Phe Tyr Ile Ala His Val Leu
                195                 200                 205
atc atc cca gct atc ctg ctt ggc ttg atc gca gct cac ctg gca ctt 3012
Ile Ile Pro Ala Ile Leu Leu Gly Leu Ile Ala Ala His Leu Ala Leu
            210                 215                 220
gtt tgg tac cag aag cac acc cag ttc cca ggc gct ggc cgc act gag 3060
Val Trp Tyr Gln Lys His Thr Gln Phe Pro Gly Ala Gly Arg Thr Glu
        225                 230                 235
aac aac gtg gtc ggt atc cga atc atg cct ctg ttc gca gtt aag gct 3108

Asn Asn Val Val Gly Ile Arg Ile Met Pro Leu Phe Ala Val Lys Ala
    240                 245                 250
gtt gct ttc ggc ctc atc gtc ttc ggc ttc ctc gca ctg ctt gct ggt    3156
Val Ala Phe Gly Leu Ile Val Phe Gly Phe Leu Ala Leu Leu Ala Gly
255                 260                 265                 270
gtc acc acc att aac gca att tgg aat ctt gga ccg tac aac cct tca    3204
Val Thr Thr Ile Asn Ala Ile Trp Asn Leu Gly Pro Tyr Asn Pro Ser
                275                 280                 285
cag gtg tct gct ggt tcc cag cct gac gtt tac atg ctg tgg aca gat    3252
Gln Val Ser Ala Gly Ser Gln Pro Asp Val Tyr Met Leu Trp Thr Asp
            290                 295                 300
ggt gct gct cgt gtc atg ccg gca tgg gag ctc tac ctc ggt aac tac    3300
Gly Ala Ala Arg Val Met Pro Ala Trp Glu Leu Tyr Leu Gly Asn Tyr
        305                 310                 315
acc atc cca gca gtc ttc tgg gtt gct gtg atg ctg ggt atc ctc gtg    3348
Thr Ile Pro Ala Val Phe Trp Val Ala Val Met Leu Gly Ile Leu Val
    320                 325                 330
gtt ctg ctt gtg act tac cca ttc att gag cgt aag ttc act ggc gac    3396
Val Leu Leu Val Thr Tyr Pro Phe Ile Glu Arg Lys Phe Thr Gly Asp
335                 340                 345                 350
gat gca cac cac aac ttg ctg cag cgt cct cgc gat gtt cca gtc cgc    3444
Asp Ala His His Asn Leu Leu Gln Arg Pro Arg Asp Val Pro Val Arg
                355                 360                 365
acc tca ctc ggt gtc atg gcg ctt gtc ttc tac atc ctg ctt acc gtt    3492
Thr Ser Leu Gly Val Met Ala Leu Val Phe Tyr Ile Leu Leu Thr Val
            370                 375                 380
tct ggt ggt aac gat gtt tac gca atg cag ttc cat gtt tca ctg aac    3540
Ser Gly Gly Asn Asp Val Tyr Ala Met Gln Phe His Val Ser Leu Asn
        385                 390                 395
gcg atg acc tgg atc ggt cgt atc ggc ctc atc gtt gga cca gct att    3588
Ala Met Thr Trp Ile Gly Arg Ile Gly Leu Ile Val Gly Pro Ala Ile
    400                 405                 410
gca tac ttc atc act tac cga ctg tgc atc ggc ttg cag cgc tct gac    3636
Ala Tyr Phe Ile Thr Tyr Arg Leu Cys Ile Gly Leu Gln Arg Ser Asp
415                 420                 425                 430
cgc gag gtc ctg gag cac ggc atc gag acc ggt atc atc aag cag atg    3684
Arg Glu Val Leu Glu His Gly Ile Glu Thr Gly Ile Ile Lys Gln Met
                435                 440                 445
cca aat ggt gcc ttc att gaa gtt cac cag cca ctt ggc cca gtt gat    3732
Pro Asn Gly Ala Phe Ile Glu Val His Gln Pro Leu Gly Pro Val Asp
            450                 455                 460

gac cat ggt cac cca atc cca ctg cca tac gct ggc gct gcg gtt cca    3780
Asp His Gly His Pro Ile Pro Leu Pro Tyr Ala Gly Ala Ala Val Pro
        465                 470                 475
aag cag atg aac cag ctt ggt tac gct gag gtt gaa acc cgc ggt gga    3828
Lys Gln Met Asn Gln Leu Gly Tyr Ala Glu Val Glu Thr Arg Gly Gly
    480                 485                 490
ttc ttc gga cct gat cca gaa gac atc cgt gcg aag gct aag gaa att    3876
Phe Phe Gly Pro Asp Pro Glu Asp Ile Arg Ala Lys Ala Lys Glu Ile
495                 500                 505                 510
gag cac gca aac cac att gag gaa gcg aac act ctt cgt gca ctc aac    3924
Glu His Ala Asn His Ile Glu Glu Ala Asn Thr Leu Arg Ala Leu Asn
                515                 520                 525
gag gca aac att gag cgt gac aag aat gag ggc aag aac tagtttctag    3973
Glu Ala Asn Ile Glu Arg Asp Lys Asn Glu Gly Lys Asn
            530                 535
gacttcatct ctgaaactcc ccgctgtagg gacctgaatc gaaaggtctc cgcatcgggg 4033
agtttttctc tattcagacg aggctaaaga taagggagag ggctctttaa cacacgagga 4093
gtggcgtaga gcctgtagtt gccttatatg tagcttgtgg cggcgtgaag caacgtgcag 4153
gcgcgtggaa agcgtagagt tttcttctcc ttatatataa ggagtgttct tcgtcgtgag 4213
cattacgccc taggtcgcct ggggtcggtg tccaacttcg ctcaaattcc gctcaaaatc 4273
cagctcggtg tggcttaaga attgttgtca taactccaaa tctcaaagca taagccgtca 4333
atggtgatta atgtcacatg gtgagatcat tgctgaaact ggtgccgatt tttcggctct 4393
gtgaaaacga tttgactact ggaagtttcc tgaaattgca ggtcatctag ctttctcagg 4453
gttctagggg agaaccctta gtggttgggg tctgagtgga ggacttgcgt ctcggtcaaa 4513
ttaatccgcg ataacggttc gataacgacc aattttttcg cttgggctag acaagtgttg 4573
ttgcggtttc gtaaccttat tgagacattg cgggacggac accgaatttc cgccagcatt 4633
acagaaacaa atagacgctt aatcgcaagc atagtttaga gaaattcttt            4683

<210> 10
<211> 539
<212> PRT
<213> Corynebacterium glutamicum

<400> 10
Met Ser Leu Ala Thr Val Gly Asn Asn Leu Asp Ser Arg Tyr Thr Met
1               5                   10                  15
Ala Ser Gly Ile Arg Arg Gln Ile Asn Lys Val Phe Pro Thr His Trp
            20                  25                  30
Ser Phe Met Leu Gly Glu Ile Ala Leu Tyr Ser Phe Ile Val Leu Leu
        35                  40                  45
Leu Thr Gly Val Tyr Leu Thr Leu Phe Phe Asp Pro Ser Ile Thr Lys

    50                  55                  60
Val Ile Tyr Asp Gly Gly Tyr Leu Pro Leu Asn Gly Val Glu Met Ser
65                  70                  75                  80
Arg Ala Tyr Ala Thr Ala Leu Asp Ile Ser Phe Glu Val Arg Gly Gly
                85                  90                  95
Leu Phe Ile Arg Gln Met His His Trp Ala Ala Leu Leu Phe Val Val
            100                 105                 110
Ser Met Leu Val His Met Leu Arg Ile Phe Phe Thr Gly Ala Phe Arg
        115                 120                 125
Arg Pro Arg Glu Ala Asn Trp Ile Ile Gly Val Val Leu Ile Ile Leu
    130                 135                 140
Gly Met Ala Glu Gly Phe Met Gly Tyr Ser Leu Pro Asp Asp Leu Leu
145                 150                 155                 160
Ser Gly Val Gly Leu Arg Ile Met Ser Ala Ile Ile Val Gly Leu Pro
                165                 170                 175
Ile Ile Gly Thr Trp Met His Trp Leu Ile Phe Gly Gly Asp Phe Pro
            180                 185                 190
Ser Asp Leu Met Leu Asp Arg Phe Tyr Ile Ala His Val Leu Ile Ile
        195                 200                 205
Pro Ala Ile Leu Leu Gly Leu Ile Ala Ala His Leu Ala Leu Val Trp
    210                 215                 220
Tyr Gln Lys His Thr Gln Phe Pro Gly Ala Gly Arg Thr Glu Asn Asn
225                 230                 235                 240
Val Val Gly Ile Arg Ile Met Pro Leu Phe Ala Val Lys Ala Val Ala
                245                 250                 255
Phe Gly Leu Ile Val Phe Gly Phe Leu Ala Leu Leu Ala Gly Val Thr
            260                 265                 270
Thr Ile Asn Ala Ile Trp Asn Leu Gly Pro Tyr Asn Pro Ser Gln Val
        275                 280                 285
Ser Ala Gly Ser Gln Pro Asp Val Tyr Met Leu Trp Thr Asp Gly Ala
    290                 295                 300
Ala Arg Val Met Pro Ala Trp Glu Leu Tyr Leu Gly Asn Tyr Thr Ile
305                 310                 315                 320
Pro Ala Val Phe Trp Val Ala Val Met Leu Gly Ile Leu Val Val Leu
                325                 330                 335
Leu Val Thr Tyr Pro Phe Ile Glu Arg Lys Phe Thr Gly Asp Asp Ala
            340                 345                 350
His His Asn Leu Leu Gln Arg Pro Arg Asp Val Pro Val Arg Thr Ser
        355                 360                 365
Leu Gly Val Met Ala Leu Val Phe Tyr Ile Leu Leu Thr Val Ser Gly
    370                 375                 380

Gly Asn Asp Val Tyr Ala Met Gln Phe His Val Ser Leu Asn Ala Met
385                 390                 395                 400
Thr Trp Ile Gly Arg Ile Gly Leu Ile Val Gly Pro Ala Ile Ala Tyr
                405                 410                 415
Phe Ile Thr Tyr Arg Leu Cys Ile Gly Leu Gln Arg Ser Asp Arg Glu
            420                 425                 430
Val Leu Glu His Gly Ile Glu Thr Gly Ile Ile Lys Gln Met Pro Asn
        435                 440                 445
Gly Ala Phe Ile Glu Val His Gln Pro Leu Gly Pro Val Asp Asp His
    450                 455                 460
Gly His Pro Ile Pro Leu Pro Tyr Ala Gly Ala Ala Val Pro Lys Gln
465                 470                 475                 480
Met Asn Gln Leu Gly Tyr Ala Glu Val Glu Thr Arg Gly Gly Phe Phe
                485                 490                 495
Gly Pro Asp Pro Glu Asp Ile Arg Ala Lys Ala Lys Glu Ile Glu His
            500                 505                 510
Ala Asn His Ile Glu Glu Ala Asn Thr Leu Arg Ala Leu Asn Glu Ala
        515                 520                 525
Asn Ile Glu Arg Asp Lys Asn Glu Gly Lys Asn
    530                 535

<210> 11
<211> 20
<212> DNA
<213> Artificial Sequence

<220>
<223> Description of Artificial Sequence: primer for PCR

<220>
<221> misc_feature
<222> (8,14)
<223> n=a or g or c or t

<400> 11
tcatggtntg ggyncaycay                                                20

<210> 12
<211> 20
<212> DNA
<213> Artificial Sequence

<220>
<223> Description of Artificial Sequence: primer for PCR

<220>
<221> misc_feature
<222> (18)
<223> n=a or g or c or t

<400> 12
ataacrtwrt graartgngc                                                    20

<210> 13
<211> 21
<212> PRT
<213> Corynebacterium glutamicum

<400> 13
Glu Val Ala Pro Pro Gly Gly Val Leu Gly Asp Phe Leu Arg Met Gly
  1               5                  10                  15
Trp Pro Asp Gly Ile
             20

<210> 14
<211> 20
<212> DNA
<213> Artificial Sequence

<220>
<223> Description of Artificial Sequence: primer for PCR

<220>
<221> misc_feature
<222> (15)
<223> n=a or g or c or t

<400> 14
ggygayttcy tbcgnatggg                                                    20

<210> 15
<211> 20

<212> DNA
<213> Artificial Sequence

<220>
<223> Description of Artificial Sequence: primer for PCR

<220>
<221> misc_feature
<222> (15)
<223> n=a or g or c or t

<400> 15
ggaccgcasa rytcngmrca                                                    20

<210> 16
<211> 16
<212> PRT
<213> Corynebacterium glutamicum

<220>
<221> unsure
<222> (1)
<223> Xaa=unknown

<400> 16
Gln Ala Glu Arg Lys Ala Pro Arg Ile Thr Glu Ala Gln Val Leu Ala
  1               5                  10                  15

<210> 17
<211> 24
<212> PRT
<213> Corynebacterium glutamicum

<400> 17
Leu Arg Gly Glu Asn Tyr Asp Gly Gln Ile Thr Ser Ala Asp Val Ala
  1               5                  10                  15
Arg Gly Gly Asp Leu Phe Arg Leu
             20